# EUROPÄISCHE PATENTANMELDUNG

(11) **EP 1 249 495 A1**
(43) Veröffentlichungstag der Anmeldung: **16.10.2002**
(21) Anmeldenummer: 01109024.8
(22) Anmeldetag: 11.04.2001
(51) Int. Cl.: C12N 15/55, C12N 9/22, C07K 16/40, C12Q 1/68, G01N 33/577, A61K 31/713, A01K 67/027

(54) **Murine DNase X, diese enthaltendes Arzneimittel und nicht-menschliches Säugetier mit verändertem DNase X-Gen**

(71) Anmelder: Deutsches Krebsforschungszentrum Stiftung des öffentlichen Rechts, 69120 Heidelberg (DE)
(72) Erfinder: Die Erfindernennung liegt noch nicht vor
(74) Vertreter: Schüssler, Andrea, Dr.

(57) **Zusammenfassung**

Beschrieben werden eine DNA-Sequenz, die murine DNase X kodiert, das murine DNase X-Protein sowie anti-DNase X-Antikörper. Beschrieben werden auch die vorstehenden Verbindungen enthaltende Arzneimittel, die vorzugsweise zur Prävention und/oder Behandlung von Erkrankungen, bei denen Apoptose eine Rolle spielt, eingesetzt werden, sowie auf diesen Verbindungen basierende Diagnoseverfahren und Kits. Schließlich wird auch ein nichtmenschliches Säugetier beschrieben, bei dem das DNase X-Gen verändert, vorzugsweise inaktiviert ist.

SEQUENZPROTOKOLL

<110> Deutsches Krebsforschungszentrum

<120> Murine DNaseX, diese enthaltendes Arzneimittel und
      nicht-menschliches Säugetier mit verändertem DNaseX-Gen

<130> K2915

<140> EP 01109024.8
<141> 2001-04-11

<160> 7

<170> PatentIn Ver. 2.1

<210> 1
<211> 1527
<212> DNA
<213> Mus musculus

<220>
<221> CDS
<222> (265)..(1149)

<400> 1
   gatagagcat ttgcctagta ttcatgtggt accagaacta acaaatttct gccatgaggc 60
   atttttctaa actgccactc cagtggggac ctgaacaggt ggtcaagctc tgtgtacaga 120
   agttgaaata cgttccagga agagattcaa gaaatgttca gggccaacag cagggagaat 180
   actaattagc tgagatcagc tgcagtgcca atgccctttg gacaacctgg attcctttgg 240
   agggttcctg atgcacacat agca atg cgt ggc ctt gtt atg gct ccc ctg    291
                              Met Arg Gly Leu Val Met Ala Pro Leu
                                1               5
   ctc att ctc ctg gtt ggt ggg act gaa gcc ttt cgt atc tgt gcc ttc    339
   Leu Ile Leu Leu Val Gly Gly Thr Glu Ala Phe Arg Ile Cys Ala Phe
    10                  15                  20                  25
   aat gcc cac aga ctg aca ctg gcc aag cta acc aag gag tca gtg atg    387
   Asn Ala His Arg Leu Thr Leu Ala Lys Leu Thr Lys Glu Ser Val Met
                    30                  35                  40
   gat acc tta gtt cag atc cta gcc cga tgt gat atc atg gtg ctt cag    435
   Asp Thr Leu Val Gln Ile Leu Ala Arg Cys Asp Ile Met Val Leu Gln
                45                  50                  55
   gag gta gta gat tct tcc cag aat act gtc ccc ttt ctg ctt caa aaa    483
   Glu Val Val Asp Ser Ser Gln Asn Thr Val Pro Phe Leu Leu Gln Lys
            60                  65                  70
   ctt aaa agt tcc agg agt tac agc ttc cta aac agc tca ttg ctg ggg    531
   Leu Lys Ser Ser Arg Ser Tyr Ser Phe Leu Asn Ser Ser Leu Leu Gly
        75                  80                  85
   cgc agc aca tac aag gaa aag tat gtg tat atc tac agg tct gac aag    579
   Arg Ser Thr Tyr Lys Glu Lys Tyr Val Tyr Ile Tyr Arg Ser Asp Lys
    90                  95                 100                 105
   aca cag gtc cta aat ttc tac caa tac aac gac aca gat gat att ttt    627
   Thr Gln Val Leu Asn Phe Tyr Gln Tyr Asn Asp Thr Asp Asp Ile Phe
                   110                 115                 120
   gcc cga gaa cca ttt gtg gcc cat ttc act ctc cct agc aaa act ctt     675
   Ala Arg Glu Pro Phe Val Ala His Phe Thr Leu Pro Ser Lys Thr Leu
               125                 130                 135
   cca agt gtg gtg ctg gtt cct ctc cac acc act ccc aag gat gtt gag     723
   Pro Ser Val Val Leu Val Pro Leu His Thr Thr Pro Lys Asp Val Glu
           140                 145                 150
   aag gag ctg aat gcc ctc tat gat gtg ttt ctg gat gtc tac cag cgc     771
   Lys Glu Leu Asn Ala Leu Tyr Asp Val Phe Leu Asp Val Tyr Gln Arg
       155                 160                 165
   tgg cag aat gag gtg att ctg ctt gga gac ttc aat gca gac tgt gca     819
   Trp Gln Asn Glu Val Ile Leu Leu Gly Asp Phe Asn Ala Asp Cys Ala
   170                 175                 180                 185
   tcg ctg aca aaa aaa cgt ctg aaa agc ctg cta ctc cgg act aag gca     867
   Ser Leu Thr Lys Lys Arg Leu Lys Ser Leu Leu Leu Arg Thr Lys Ala
                   190                 195                 200
   ggc ttc cac tgg gtg att cct gat ggg gag gat act aca gtg cgg gcc     915
   Gly Phe His Trp Val Ile Pro Asp Gly Glu Asp Thr Thr Val Arg Ala
               205                 210                 215
   agt act aac tgt acc tat gac cga att gtg gtg cat gga cag ggc tgc     963
   Ser Thr Asn Cys Thr Tyr Asp Arg Ile Val Val His Gly Gln Gly Cys
           220                 225                 230
   caa atg ctg ctg aag gct gca gct acc ttt gac ttt ccc aag agg ttt     1011
   Gln Met Leu Leu Lys Ala Ala Ala Thr Phe Asp Phe Pro Lys Arg Phe
       235                 240                 245
   cag ctg act gag gaa gag gct ctc cgc atc agt gac cat tat cct gta     1059
   Gln Leu Thr Glu Glu Glu Ala Leu Arg Ile Ser Asp His Tyr Pro Val
   250                 255                 260                 265
   gaa gtg gaa ctg agc cag gcc act cca ctg agt atc cca ccc cac tac     1107
   Glu Val Glu Leu Ser Gln Ala Thr Pro Leu Ser Ile Pro Pro His Tyr
                   270                 275                 280
   tta gcc gct cta ctg ctg tca ctc ctg cca tct cag ctg gac             1149
   Leu Ala Ala Leu Leu Leu Ser Leu Leu Pro Ser Gln Leu Asp
               285                 290                 295
   tagatagcca ttgacttgac cagggcactg gtagtctcag atgtgaaagt caatgacagc 1209
   ttttttaaat agctgcttgc tattctgaga aagctagggc tgggagtata actcagcaac 1269
   aagtgccttg ggcttgaatc ctgttgggta gacaggactt cacctgaggt agttaggaac 1329
   aaagctccag tagaaatggg ctcagatcat acctaagatt tctgcaactg atacaaatga 1389
   gctcatactg gaaaaacctt aggtctaagt aatcttgctt ctccatgcac tatactacat 1449
   ggctctgcca gtgttgtcag gtttaaaata ttcaataaaa tcattttaac ctttaaaaaa 1509
   aaaaaaaaaa aaaaaaaa                                                1527

<210> 2
<211> 295
<212> PRT
<213> Mus musculus

<400> 2
Met Arg Gly Leu Val Met Ala Pro Leu Leu Ile Leu Leu Val Gly Gly
1               5                   10                  15

Thr Glu Ala Phe Arg Ile Cys Ala Phe Asn Ala His Arg Leu Thr Leu
            20                  25                  30

Ala Lys Leu Thr Lys Glu Ser Val Met Asp Thr Leu Val Gln Ile Leu
        35                  40                  45

Ala Arg Cys Asp Ile Met Val Leu Gln Glu Val Val Asp Ser Ser Gln
    50                  55                  60

Asn Thr Val Pro Phe Leu Leu Gln Lys Leu Lys Ser Ser Arg Ser Tyr
65                  70                  75                  80

Ser Phe Leu Asn Ser Ser Leu Leu Gly Arg Ser Thr Tyr Lys Glu Lys
                85                  90                  95

Tyr Val Tyr Ile Tyr Arg Ser Asp Lys Thr Gln Val Leu Asn Phe Tyr
            100                 105                 110

Gln Tyr Asn Asp Thr Asp Asp Ile Phe Ala Arg Glu Pro Phe Val Ala
        115                 120                 125

His Phe Thr Leu Pro Ser Lys Thr Leu Pro Ser Val Val Leu Val Pro
    130                 135                 140

Leu His Thr Thr Pro Lys Asp Val Glu Lys Glu Leu Asn Ala Leu Tyr
145                 150                 155                 160

Asp Val Phe Leu Asp Val Tyr Gln Arg Trp Gln Asn Glu Val Ile Leu
                165                 170                 175

Leu Gly Asp Phe Asn Ala Asp Cys Ala Ser Leu Thr Lys Lys Arg Leu
            180                 185                 190

Lys Ser Leu Leu Leu Arg Thr Lys Ala Gly Phe His Trp Val Ile Pro
        195                 200                 205

Asp Gly Glu Asp Thr Thr Val Arg Ala Ser Thr Asn Cys Thr Tyr Asp
    210                 215                 220

Arg Ile Val Val His Gly Gln Gly Cys Gln Met Leu Leu Lys Ala Ala
225                 230                 235                 240

Ala Thr Phe Asp Phe Pro Lys Arg Phe Gln Leu Thr Glu Glu Glu Ala
                245                 250                 255

Leu Arg Ile Ser Asp His Tyr Pro Val Glu Val Glu Leu Ser Gln Ala
            260                 265                 270

Thr Pro Leu Ser Ile Pro Pro His Tyr Leu Ala Ala Leu Leu Leu Ser
        275                 280                 285

Leu Leu Pro Ser Gln Leu Asp
    290                 295


<210> 3
<211> 1608
<212> DNA
<213> Mus musculus

<220>
<221> CDS
<222> (345)..(1229)

<400> 3
   tgtggagccg gcccctttgg gtgtgtctgg aaacccgcgc ctcttcttcc catttccccc 60
   acacacacac ccaactgcgc gtccttaccc gccttggctg gagctgcgat tctgtggagt 120
   ggaatgtttt attcggttca acgctgaggc aggcgtgggg ccgacttggg ctgtggaggt 180
   ggtcaagctc tgtgtacaga agttgaaata cgttccagga agagattcaa gaaatgttca 240
   gggccaacag cagggagaat actaattagc tgagatcagc tgcagtgcca atgccctttg 300
   gacaacctgg attcctttgg agggttcctg atgcacacat agca atg cgt ggc ctt 356
   Met Arg Gly Leu
   1
   gtt atg gct ccc ctg ctc att ctc ctg gtt ggt ggg act gaa gcc ttt 404
   Val Met Ala Pro Leu Leu Ile Leu Leu Val Gly Gly Thr Glu Ala Phe
   5 10 15 20
   cgt atc tgt gcc ttc aat gcc cac aga ctg aca ctg gcc aag cta acc 452
   Arg Ile Cys Ala Phe Asn Ala His Arg Leu Thr Leu Ala Lys Leu Thr
   25 30 35
   aag gag tca gtg atg gat acc tta gtt cag atc cta gcc cga tgt gat 500
   Lys Glu Ser Val Met Asp Thr Leu Val Gln Ile Leu Ala Arg Cys Asp
   40 45 50
   atc atg gtg ctt cag gag gta gta gat tct tcc cag aat act gtc ccc 548
   Ile Met Val Leu Gln Glu Val Val Asp Ser Ser Gln Asn Thr Val Pro
   55 60 65
   ttt ctg ctt caa aaa ctt aaa agt tcc agg agt tac agc ttc cta aac 596
   Phe Leu Leu Gln Lys Leu Lys Ser Ser Arg Ser Tyr Ser Phe Leu Asn
   70 75 80
   agc tca ttg ctg ggg cgc agc aca tac aag gaa aag tat gtg tat atc 644
   Ser Ser Leu Leu Gly Arg Ser Thr Tyr Lys Glu Lys Tyr Val Tyr Ile
   85 90 95 100
   tac agg tct gac aag aca cag gtc cta aat ttc tac caa tac aac gac 692
   Tyr Arg Ser Asp Lys Thr Gln Val Leu Asn Phe Tyr Gln Tyr Asn Asp
   105 110 115
   aca gat gat att ttt gcc cga gaa cca ttt gtg gcc cat ttc act ctc 740
   Thr Asp Asp Ile Phe Ala Arg Glu Pro Phe Val Ala His Phe Thr Leu
   120 125 130
   cct agc aaa act ctt cca agt gtg gtg ctg gtt cct ctc cac acc act 788
   Pro Ser Lys Thr Leu Pro Ser Val Val Leu Val Pro Leu His Thr Thr
   135 140 145
   ccc aag gat gtt gag aag gag ctg aat gcc ctc tat gat gtg ttt ctg 836
   Pro Lys Asp Val Glu Lys Glu Leu Asn Ala Leu Tyr Asp Val Phe Leu
   150 155 160
   gat gtc tac cag cgc tgg cag aat gag gtg att ctg ctt gga gac ttc 884
   Asp Val Tyr Gln Arg Trp Gln Asn Glu Val Ile Leu Leu Gly Asp Phe
   165 170 175 180
   aat gca gac tgt gca tcg ctg aca aaa aaa cgt ctg aaa agc ctg cta 932
   Asn Ala Asp Cys Ala Ser Leu Thr Lys Lys Arg Leu Lys Ser Leu Leu
   185 190 195
   ctc cgg act aag gca ggc ttc cac tgg gtg att cct gat ggg gag gat 980
   Leu Arg Thr Lys Ala Gly Phe His Trp Val Ile Pro Asp Gly Glu Asp
   200 205 210
   act aca gtg cgg gcc agt act aac tgt acc tat gac cga att gtg gtg 1028
   Thr Thr Val Arg Ala Ser Thr Asn Cys Thr Tyr Asp Arg Ile Val Val
   215 220 225
   cat gga cag ggc tgc caa atg ctg ctg aag gct gca gct acc ttt gac    1076
   His Gly Gln Gly Cys Gln Met Leu Leu Lys Ala Ala Ala Thr Phe Asp
       230                 235                 240
   ttt ccc aag agg ttt cag ctg act gag gaa gag gct ctc cgc atc agt    1124
   Phe Pro Lys Arg Phe Gln Leu Thr Glu Glu Glu Ala Leu Arg Ile Ser
   245                 250                 255                 260
   gac cat tat cct gta gaa gtg gaa ctg agc cag gcc act cca ctg agt    1172
   Asp His Tyr Pro Val Glu Val Glu Leu Ser Gln Ala Thr Pro Leu Ser
                   265                 270                 275
   atc cca ccc cac tac tta gcc gct cta ctg ctg tca ctc ctg cca tct    1220
   Ile Pro Pro His Tyr Leu Ala Ala Leu Leu Leu Ser Leu Leu Pro Ser
               280                 285                 290
   cag ctg gac tagatagcca ttgacttgac cagggcactg gtagtctcag            1269
   Gln Leu Asp
           295
   atgtgaaagt caatgacagc ttttttaaat agctgcttgc tattctgaga aagctagggc 1329
   tgggagtata actcagcaac aagtgccttg ggcttgaatc ctgttgggta gacaggactt 1389
   cacctgaggt agttaggaac aaagctccag tagaaatggg ctcagatcat acctaagatt 1449
   tctgcaactg atacaaatga gctcatactg gaaaaacctt aggtctaagt aatcttgctt 1509
   ctccatgcac tatactacat ggctctgcca gtgttgtcag gtttaaaata ttcaataaaa 1569
   tcattttaac ctttaaaaaa aaaaaaaaaa aaaaaaaaa                         1608

<210> 4
<211> 295
<212> PRT
<213> Mus musculus

<400> 4
Met Arg Gly Leu Val Met Ala Pro Leu Leu Ile Leu Leu Val Gly Gly
  1               5                  10                  15

Thr Glu Ala Phe Arg Ile Cys Ala Phe Asn Ala His Arg Leu Thr Leu
             20                  25                  30

Ala Lys Leu Thr Lys Glu Ser Val Met Asp Thr Leu Val Gln Ile Leu
         35                  40                  45

Ala Arg Cys Asp Ile Met Val Leu Gln Glu Val Val Asp Ser Ser Gln
     50                  55                  60

Asn Thr Val Pro Phe Leu Leu Gln Lys Leu Lys Ser Ser Arg Ser Tyr
 65                  70                  75                  80

Ser Phe Leu Asn Ser Ser Leu Leu Gly Arg Ser Thr Tyr Lys Glu Lys
                 85                  90                  95

Tyr Val Tyr Ile Tyr Arg Ser Asp Lys Thr Gln Val Leu Asn Phe Tyr
            100                 105                 110

Gln Tyr Asn Asp Thr Asp Asp Ile Phe Ala Arg Glu Pro Phe Val Ala
        115                 120                 125

His Phe Thr Leu Pro Ser Lys Thr Leu Pro Ser Val Val Leu Val Pro
    130                 135                 140

Leu His Thr Thr Pro Lys Asp Val Glu Lys Glu Leu Asn Ala Leu Tyr
145                 150                 155                 160

Asp Val Phe Leu Asp Val Tyr Gln Arg Trp Gln Asn Glu Val Ile Leu
                165                 170                 175

Leu Gly Asp Phe Asn Ala Asp Cys Ala Ser Leu Thr Lys Lys Arg Leu
            180                 185                 190

Lys Ser Leu Leu Leu Arg Thr Lys Ala Gly Phe His Trp Val Ile Pro
        195                 200                 205

Asp Gly Glu Asp Thr Thr Val Arg Ala Ser Thr Asn Cys Thr Tyr Asp
    210                 215                 220

Arg Ile Val Val His Gly Gln Gly Cys Gln Met Leu Leu Lys Ala Ala
225                 230                 235                 240

Ala Thr Phe Asp Phe Pro Lys Arg Phe Gln Leu Thr Glu Glu Glu Ala
                245                 250                 255

Leu Arg Ile Ser Asp His Tyr Pro Val Glu Val Glu Leu Ser Gln Ala
            260                 265                 270

Thr Pro Leu Ser Ile Pro Pro His Tyr Leu Ala Ala Leu Leu Leu Ser
        275                 280                 285

Leu Leu Pro Ser Gln Leu Asp
    290                 295

<210> 5
<211> 1260
<212> DNA
<213> Mus musculus

<220>
<221> CDS
<222> (688)..(870)

<400> 5
tgtggagccg gcccctttgg gtgtgtctgg aaacccgcgc ctcttcttcc catttccccc 60

acacacacac ccaactgcgc gtccttaccc gccttggctg gagctgcgat tctgtggagt 120

ggaatgtttt attcggttca acgctgaggc aggcgtgggg ccgacttggg ctgtggaggt 180

ggtcaagctc tgtgtacaga agttgaaata cgttccagga gttacagctt cctaaacagc 240

tcattgctgg ggcgcagcac atacaaggaa aagtatgtgt atatctacag gtctgacaag 300

acacaggtcc taaatttcta ccaatacaac gacacagatg atatttttgc ccgagaacca 360

tttgtggccc atttcactct ccctagcaaa actcttccaa gtgtggtgct ggttcctctc 420

cacaccactc ccaaggatgt tgagaaggag ctgaatgccc tctatgatgt gtttctggat 480

gtctaccagc gctggcagaa tgaggtgatt ctgcttggag acttcaatgc agactgtgca 540

tcgctgacaa aaaaacgtct gaaaagcctg ctactccgga ctaaggcagg cttccactgg 600

gtgattcctg atggggagga tactacagtg cgggccagta ctaactgtac ctatgaccga 660

attgtggtgc atggacaggg ctgccaa atg ctg ctg aag gct gca gct acc ttt 714
                              Met Leu Leu Lys Ala Ala Ala Thr Phe
                              1               5

gac ttt ccc aag agg ttt cag ctg act gag gaa gag gct ctc cgc atc 762
Asp Phe Pro Lys Arg Phe Gln Leu Thr Glu Glu Glu Ala Leu Arg Ile
10                  15                  20                  25

agt gac cat tat cct gta gaa gtg gaa ctg agc cag gcc act cca ctg 810
Ser Asp His Tyr Pro Val Glu Val Glu Leu Ser Gln Ala Thr Pro Leu
                30                  35                  40

agt atc cca ccc cac tac tta gcc gct cta ctg ctg tca ctc ctg cca 858
Ser Ile Pro Pro His Tyr Leu Ala Ala Leu Leu Leu Ser Leu Leu Pro
            45                  50                  55

tct cag ctg gac tagatagcca ttgacttgac cagggcactg gtagtctcag 910
Ser Gln Leu Asp
        60

atgtgaaagt caatgacagc ttttttaaat agctgcttgc tattctgaga aagctagggc 970

tgggagtata actcagcaac aagtgccttg ggcttgaatc ctgttgggta gacaggactt 1030

cacctgaggt agttaggaac aaagctccag tagaaatggg ctcagatcat acctaagatt 1090

tctgcaactg atacaaatga gctcatactg gaaaaacctt aggtctaagt aatcttgctt 1150

ctccatgcac tatactacat ggctctgcca gtgttgtcag gtttaaaata ttcaataaaa 1210

tcattttaac ctttaaaaaa aaaaaaaaaa aaaaaaaaaa aaaaaaaaaa 1260

<210> 6
<211> 61
<212> PRT
<213> Mus musculus

<400> 6
Met Leu Leu Lys Ala Ala Ala Thr Phe Asp Phe Pro Lys Arg Phe Gln
1               5                   10                  15

Leu Thr Glu Glu Glu Ala Leu Arg Ile Ser Asp His Tyr Pro Val Glu
            20                  25                  30

Val Glu Leu Ser Gln Ala Thr Pro Leu Ser Ile Pro Pro His Tyr Leu
        35                  40                  45

Ala Ala Leu Leu Leu Ser Leu Leu Pro Ser Gln Leu Asp
    50                  55                  60

<210> 7
<211> 40116
<212> DNA
<213> Mus musculus

<400> 7
gatcctcggt ataaacaagt gctcttatct gctgagccat ttctccagcc tctactctgc 60
tttttaatgc actagcgcag tgcccccatt acccagggat ggaataatat acatgaaaat 120
tagatgggaa agtaggagat gcctcaaaaa tattcaaggt aattttcatt cttggcctta 180
gtcttactag taatactatc cgaaaaagac agatcctcac ctggcaaagc cctggggcag 240
ctcacccagg ccatacagtg ggtataaata ggggctcttg ccataccggg ccagggactc 300
gctgtacaac ttgatgcggt taatagtctc aagacagggc tgatccaggt agctggggaa 360
caagagccaa ggaagatgct ttggcagcgg catccttgct cacctacaaa gacaccagcc 420
aaccttccca ccaccctttg tgacctggca aagatgtggg tcaaatggag agggactgaa 480
atggaacagg tgagtctgga gctgagcaca agagacatgc agggtgggca gtaggtacaa 540
taggcaggtg tggggagggc aaggtgtgag ccagacaagg ggctcccatc accagccagc 600
tttcccctca ctcatcagtg cggtagagcg ccaaggcatg gccagtgaaa tctatgacat 660
cttgacccag gctctggctc ctggtagaca tcacccatgc tggtgttctg gggatcaaca 720

ccctcaaagg ttttggggtc attctcatca aagtttgcca caaacaccaa aaattttcgg 780
aagcgtcgtt tttcaaacat gcccatcaga tctgaaagaa aggtattgag aagaccctta 840
gcattactgg gacatagaac tcctctttcc accagagcag gccaagaaag ctggttgtat 900
gtaatgtaga gtattggctc ccaccaatac tctatcatgt aacactaccc cacccttctt 960
gatctcaatt ttctttttgt aggaagaaac ccttctagtt ccacgtaggg atgctacagg 1020
gcaagtaggc cctctgacaa aaaggaatcc tgttctgtga actttctggc aacttgccag 1080
gatgggtagc actgtccaat caatacacta taaagtaagg gtcctctagg actacacaga 1140
gctaagatta gtttcaggca aagatgagcc ttttgcctaa ctgtcctgaa aatagtcgtc 1200
tcatccagcc ccattattct ctggttaagg agactcatac tcactagaag ccaaggcctc 1260
agtctcggtg gatggcaatt tgtagatcct gcccccctta tatacaaagg tgccctctac 1320
caccttgaag tccagatatc gagtcacttc tgtatatagt agcatcttta ccagctgacc 1380
tgtgtccaga ggaaataagg gacattgctg tgttttcctt caaaaactct cagtagtctg 1440
actgaccata ttcttgagat tgaccaatct ctccccactc cgtcttccca ctcaccaccc 1500
ccaaaaccat atacagcatc tttttaatct ctctcaccat tggccatgag gaatttgggg 1560
atcaagtcaa cattccagtc tcggacccgg cccattgatt cagggggccc ttccagtatc 1620
tgaaagcgct tatatagctg taagcaggac aggaaagaaa gaaaaatgtc aaggacctgt 1680
cgccctttcc cactctcctg cccagtacct gagaattact tcgccccctt atacctatgt 1740
ggaagactaa caaggatctg ggtctaagcc caggtctaag aataagaaat cttacctcct 1800
ccaggggtgt gatggaagag ctctcacccc catagtaggg gtttcggtcc atgtgcagca 1860
ccttctttcc attcacagac atgatgccag acaggatgca ttcctgggga aggagtgaca 1920
tttgcagcta taccccataa ccctccaact tgcttcatgc ccaccaccaa ttttataatg 1980
cagctctttt cagtgcctaa aactggggag gagaagatgg cttcatatga tgtttccttt 2040
acaaccctgc tgagtaaact actggagcat taaaggaggt ttggggggca gagctttcag 2100
cttccaagtc ttccagcatc aacaagccaa gagaagtatc tacttctctg agcccacaga 2160
aagacaatga gtctacaaga agaaccaatt caaaggacct ttgaaagaga aggccaagtc 2220
cttgaaatcc caggccccac cctgctcctc cttcaaaggg caggagcccc tgactctctt 2280
ctggccctat ccactggtct agactcaggg ttcaaactaa aaagatttat gccgggggtg 2340
ggggttgggg ggggcggggg cagggaagag acaaacaccc tcaccagtca gtcactaacc 2400
caaggacaaa ggctgaggat atatattcag tctgagatct ccatgggaaa ccaagttggg 2460
gcagaggtaa agtggtcact accccccccc acaccccccc acacacactc ctgctgtctc 2520
tggaacattc catgggtttc ctatcccatc ccaaaaagca gaaaagcatg atgaggcccc 2580
acccttgtaa tgtccccata cccatcttca tattccatcc cctagtttga tcacaagatg 2640
gtaagggatg ctaatcaagg ccttggagca ggtgatgagt tcaggcaaag ggtgggggct 2700
gggtgcaatg gttccactgt gtgtgtgggg gggtgagttt tccacctcgg tgatagagat 2760
agggaagaat aggggtctga ggttaagtac tagagtgcag gaacacagga caaagtgggg 2820
ccctctacag agaattggga actggaatcg acatcctagg gtgaggtagt gggttgggca 2880
tggggttggt tttcccaggg ctggagctag gatgatggga ggatggatcg gataccggac 2940
tctgcatcaa tgcgggatgg agtggggtgg ggagggatgg cgtggggtgg ggagggatgg 3000
catgatgcag ggctatgaga gcctgaagtg gcacttacgg taagacctgt ccccagcaca 3060
atcacatcgt attcctcatc catgatcagg ccttggtacc agcgtccgct ctgctcctcc 3120
tccttcacca cagcagccgc taccactacc tgtactattg ccactgctgc tactgccgct 3180
gctgctgctg cccctgccgc tgcttctgtc actgccgccg cactgaacca aagatggcgt 3240
ctgtcggttg tctcctgatc cctacgcagc gttaatgagt ccctctctct ctccgctggc 3300
cccgccccgc ccaaccagtc atcatttcct tgcttgtttt gtgggagggc ttctgtcacc 3360
tgggtcccag caccagaatg actgcagggg ttatcggggc tcttcgtgag cccagaaaaa 3420
tacggggcgg ggcaggccgg cagcaggtgc tgagtcctcc gaaagatcct tgcgcgggag 3480
ccgcgcgcgc cccgccccgc cccgcgcgct cccgtaccgc cccctgggag aaacacaagg 3540
tatggaagcc ctgctgtgtg ttgttgcaca gtatcggagc taccattgtc aggattcctc 3600
ccaaggccag gaaaagtaac aagcctctac tctaagtagg tttgttaaga gtatcagctt 3660
actgaagacc ctcccaccct tcctctctca cctcttgcat gactggacta catctgagca 3720
cttcttccct tcagtcaaga aaaaaaaatc tactgttcag tgcagaaatg agtgacacga 3780
agcccaacac aaggcaacac gtctttattt tattaggaag gaaacaacag agcaaccccc 3840
cacacacacc tccggcctct tgacaggcag tctttatggg aataatccag aaacccagtg 3900
agcacacttt gttcaccaat cctcgtgatg gtttcttcct tcccaggcca cagaaataaa 3960
ccccattgta ccactcactc ttaacacatc acccctcccc cccaaaaaac ataacagcta 4020
agctggacag acagtcaagt agtctttcca cacctcgcaa attcaaggaa cctcaagttc 4080
atggctgttg ctggaagctc cataaagctt ggggagtgag catttactac acaaaaccac 4140
tataaacagc atggaataat agcagagggg caagaataaa taaggagaga gggtaaaggg 4200
gagaccctac aattaaggtc ccttcccctc ttgatgatga accctggaag atttatctaa 4260
tatctatgaa caatacactt tgttggtaaa aatgggggat agccacaggg gagtccctct 4320
ccgggttaaa gaggaagcaa gagagactga gggtggcgta tgtagggttc atgctatact 4380
aggaagagga caaacctttc catccactgt gtcacaaaac agaaacatga caacctagac 4440
accaatactc cctcaattcc tctcaggtgt atgtcacaca atctgggtca aagtgatggt 4500
ggggcccttg tggtcatcaa agcgatccat ggtctttagg ctgagtatca tgtgcagacc 4560
ataggtgaat atgaaaagca tgaagagggt ggtgagcaga cccatccaga tgcccggaga 4620
gaagaaacca gcacagtcgc tagcatagga gaactgttca ccagtcacat tgaaggcctg 4680
gatctaggag acacattgac atggatcaga tggttccaca aggttcccca cacaggaaga 4740

gctgggattc tgggatcaat gaataatcta taccttcaga gactggacaa ggctggaact 4800
ctcagctaca tgctcaaacc atactgagat tctctgggcc cttcttccct ccccttactg 4860
ccaccagctt ggggcccatc accctttcaa tacctggaag ttatgaagag tcatctgcca 4920
gactgagggc acgttggtca caaggagatt gcctttcttg ctcacactgc tgacgtactc 4980
acagtggaaa gaatagatgc taggtcctgt gacttgggaa acattgaaat gggcaacaga 5040
gccattgctg tggatttcaa gtcgttccat ggcaaaccaa tatcgggcag acactggata 5100
gaagcgacta gccagaatga acctgggagg caggggatgc agagatgtca gatgaacaga 5160
acagagtgaa gacacgccag ccctgacaaa cttcagccaa cagaacttac ttgaatgtca 5220
ctgttgcacc aaagagtggc tcataggtca gtgaaagcct atggaacaag gcagaggagt 5280
actgtcaggc tctagaagga tcttaagaag gtcctcatca ggttggctcc aacctaagct 5340
gccctagatc catccttata gtcacctgcc aatacagtgt ccccatttac catatgggaa 5400
aagtaaagtt ccagtcataa gcaaatcagg agcagttgtg aaacaattag gtttcacatc 5460
tccaacttag gattccttcc tctggttcct aggcctcctt cttggggtta ttcagctgcc 5520
tgctgaggtt tcaagggtag gaaacctagg actggtttta acagttggtt tgatatttgc 5580
ctacatgcag tcccaggggt tgggttcaaa aacggtccag gatcccaagg gactatgtgg 5640
taataaaagc tcaggaccca acaaccaata cctaaaaaaa catgagaagg ccaaactctt 5700
aagaggctcc ctcaccagta acctcccagc aatatcaccc tgcgatactg tgacatctat 5760
gtccttggtg aacataccac acaaacataa tcccacatcc ttgtcctgta ctgagctcac 5820
agtgacagga agaccatgtg tgataggata gcccttcctt ctcttcctat aacccttaca 5880
tggcaaagga gtcattccag aaggagccgg tcaagttaag attctccaca ccaaaggtga 5940
gggaggtcaa gtctttccac tcgtctttgt atgccacaga gaagttttgt gcccaaaaca 6000
ggatccttgg ggcagtgtca ttgtaactca caggaggatg gatcgcagga gatgccacct 6060
gagtctgaag cagctggcga cctagacccc cagccaccat ggttatatca cgggccacct 6120
gtagaaacat ccaacaagcc ttctctcaac agccctaaaa acaaaaccta atccactgga 6180
ctggtattcc ttccccactg gttaccctgt ataccatctg aaagtcccca gacctgatat 6240
tccatctccc tagactaaca accacactgc ccaaagtggc tcataagcaa aatgaaaaaa 6300
ggcaccatgg atggaggaag caatcctctg tccattaatc ccccaaactg aagcctcctg 6360
agagatcctg cctgtgacat accccttcat ctctagtcac actcaccaca ttgcctcaca 6420
ccctcattcc ccagggcccc tgctagaaga gtacatactc tagaagggcg gactgcagta 6480
agagctgcgg tgtaagggac atcttcagac ttgagtgtgc tcagtacctg tccgatgacc 6540
tcatctgaag agtgacaaga gaacagagag ccaagtcacc aggtagcttg cttagagaaa 6600
taccacccat tgtctgattc agagaccttc tagactatgg gaattcagtg ctgttagttt 6660
ccactgagga cctctaaaaa acaggtattt ctccttctac atactctgcc agtctctctg 6720
gggacaattg tagctctctg atggcaagct aatcctaagt aaacagaaca tatagtgata 6780
cagagaactc aaaatctaca cagcagatgg gactgtcctt taccacaaag aagtactttg 6840
cataatgtta ctggtctatt aaatacagag attcattgga cttgtacaaa aactagattt 6900
tggagctctt gcacaggggg tcataaggca cttggatatc tctgtcaggt ggggctgact 6960
acagaagaac atggccaagc agccagccac gatgtacttg ctcagctgtc agccactctg 7020
agaaacgggg acaaccttgt ggtaaggaga aaccagttct attttcctgg cccaagggca 7080
aaatggggac ataaatttcc cagtaacaca ctcaccgtta cctgtgagga cctccctggg 7140
cgccatcaga ccggagctat ggataaaaaa agtgagaggc ccacaggttg ctctgctctc 7200
aaatccaaat agcttgcctg gatcagttct ctgaaactca ctacctttct ctcagccctt 7260
ctaacatttc ccttcctact agccccactt actaagtctg tggacttcat ctgctgctct 7320
gtcatgcaga cacttagctc agcaacagct attcccactt ccacacctct atttgcttca 7380
gtacccctgt ctggaatcct attccccttt cccctatttg caactatgtc tacagtccta 7440
taaagtgggc attagtggta acaaagtttt ctcatttctg gaggcccaat aggcctaact 7500
cattggtgaa tacttgtaat tcacaacctg acaggaggtc ctttctgtca cttcttggaa 7560
taaaatgaac tttctgtaag attcccaagg tattgaacag gagaatactg tcatctttgg 7620
ttgaactctg ccaccatcag ttcccaggat cttaccccta tctgaggtgc aaggttagag 7680
gctggaaccc atgccaccta ggctgtacct ggctgtttag ggcaaacggt tgaccaccag 7740
ggaaggaagg ctggcattga gcttcacctc ctttaaggta cctagttcca catgcaaggg 7800
gctaccccca ggcttctcct gtaggtaggt ggtcagagtg ctgattgcat accattccac 7860
agcaggaacc accattgagg aggggggcaaa ttcaagggaa ttctgatggt agagaagatg 7920
tggaaggcaa attgacccag ggatcagtgg ttcttacaac taaccactaa aacctcacct 7980
ggaaagccac accccatagg gactggatcc cagaatgagt cacgaaagta aatgtcccat 8040
atctccaagc cttgacagtc acatcccaag ccctctaccc tcaaagcctc tggtcctctc 8100
tctgggagag aacacagaat gtattgtctt tccttttggc tgtgagcaga cccttacctc 8160
caggttagaa aaggcactgt cctgcttatt tccaaacaca ccaccgtatg ctgtgaaatc 8220
ctcaatgctt agctaaaagg aaagcagaag agatgtccta ggaggctggg caaggaacaa 8280
agaccaaggg ccaggacatg gaggggagat tctccaggct aacaaaagag tgaaccatca 8340
ggacttgtgg tggaggtgag tggaaggctt actcttttct cccagcactc tgagggcatg 8400
atgacaagat atagaaggtg atcatagggt tgagagattt atgtacagct ggtttgtgat 8460
aacctacaaa acaaggtca ggcagcaaga agaactgggg aggaagaggg aagttagaga 8520
acaggaagag gacagcaaag aacaattact atatgcctct aaagagggaa gtacaagtca 8580
gagcaaatga acctctatac agtttgggca ggatcctttt caaaggagga agtccatgcc 8640
ccaccattgt tgttaagaat gaggggagac acgcctttaa tcccagtact cgggaggcag 8700
aggcaggcgg atttctgagt ttgaggccag cctggtctac agagtgagtt ccaggacagc 8760

cagagctaca cagaggaaac cctgtcttga aacaacaaca acagcaacaa caaaacaaca 8820
acaacaaaaa aagagtgagg ggagaggaag atattggaac aaaatggggg aaacagattt 8880
cggagctctc aggaccctgg caatgccaat ctcatgccct tctctctaca acctaaaagt 8940
gttttcactc ctccttccct caacccccag ctagcttcct ctggcttctc ccagtttagg 9000
cccagaggca atcttaacca gcttccctaa actcattatg tttactatac ataagcccag 9060
tctcctttcc tatgttcctc tcctatattc tgtgctacct atgcaaggct tgctatcttc 9120
ctaccaacct ttccccttgt ggcatttcct ccatctctag gtgacctagc ggatcatctc 9180
ctaacctttc aatcacatac taggctccag tctcacaagc agcccctaga actgattaag 9240
aacactccct tgcccagggc ccttacacct gcagttctcc tgccagtaat ggcctgcctc 9300
aaaagtcaca tgactccttc ctgccaataa gatgttatct ccagagaccc tgcctgatct 9360
cactgttacc taaaacagtg tccttactgt gttgtcttag cctgctccct tctctagact 9420
aaaaagactg gtatcataaa catgtaccaa cttagctctt agccacttgc cttcacaaga 9480
atgtttatac ttccaaacac atagcaagca cgccaccatt gtcaaaagga aatgtcctaa 9540
gattgagaga ctgtggctag atgacttatg tagaatactt ctcgcagact cctgatatag 9600
gtggatatct tcattttgca cttacagaaa agtatggtta acaaagatta tttcatgcgc 9660
tcaaaggctg aaaaatgata ggactgagtt ctggtctgac ctgttattta ccatcttctg 9720
aaaggactat caaccagcac aattactacc accgacacag gaactgaagg aaagcaagaa 9780
cagaaagagc tagtcaggct actatggagg aagggacaag cagagacttg acatcctctt 9840
gaacaataat cttatgctac attagaacct tctcatagtt catactcctg ctctaggtgc 9900
agacctctgg agtgaaagga aaatagaatg ctggatcagc acagaactga aggacaggtc 9960
tgataccctc aggctggaag tagtgtattg tgcaaatcac aattcagcaa ttcattttac 10020
ctcttcaaca ttactttcct tagcagttaa aacaagataa tgagggctgg tgagatggct 10080
cagtgggtaa gagcacccga ctgctcttcc gaaggtccga agttcaaatc cccggaacca 10140
cattgtgggt aaaaaccccc cttaatggga aattttcccc cttttggtgc attttaagac 10200
cggaccgttt acctaccttt ataaataaat aaaattttta aaaaaaaaaa aaaaaaaaaa 10260
aaaaaaaaaa aaaaaaaaaa aaaaaaacaa gataatgata tcagttgaat agaatgcaca 10320
taccagacta aataaaaggt cattttccag ttattcctat tactgccaac cattagctat 10380
tatatatata tatatactac atatatatat atatatatat atatatatat atatatatat 10440
atgaatgaat gaaatgaaac cttgggcaag tcactgatct tatcccaaaa tgagtcttct 10500
catctgtgaa actgagtagt aatttccacc acagtatagg acatcagaaa gtaatgctta 10560
gtactaggta ggtatctggt ctttaatcca ttgcttcctt ttttttttct ttttggtttt 10620
tcgagacagg gtttctctgt gtagccctgg ctgtcctgga actcactctg tagaccaggc 10680
tggcatcaaa ctcagaaatc cgcctgcctc tgcctcccaa gtgctgggat tgaaagagtg 10740
tgccaccact gcccagttaa tcccttgctt ccttaaatca agtccctgct gtttctgtgg 10800
gtctactgtt cccactgaag cttctcctac atatctttcc aaaaatctag ccacacctgc 10860
ctacaggtgg aaggaggtca ctacagcttc acctatggaa cgggcagtag gggtggggtg 10920
gggtcagctt tccttggaca aagacatagc aagctagaac agactcacct tgtcctgtag 10980
gaacagcagt acattacggg gacccagctc cagggcaggg tctaagtagg tagaaagctg 11040
catatcgctg gtgatatgac cctcatgtgt gtcggccaca ggagcccaca gattcctgga 11100
agtggacaga atagggactg tcagacctga tttcgttgct tgagatccta accctatcgg 11160
ctcctggctt cacatgcaag gccctgagat ctgaacgctc gccgtcatga agcgagtccc 11220
tgaataaaag acaatctcca tctgggcctc gaagcctctg gccaaggaca gccctggagt 11280
tcagcaaagg cgactcatcc accgcggcgc accgcatggc actcccgaac ccaacctggc 11340
ccgattcaac tcagccaagc cggccagccc aaccaggcct gtccctgccc gctcaccggt 11400
cactcgacca cagtaccagt ggcacctgct gctccgtggc cactgctgct gccactgcca 11460
ccaacgacag agacagccac atgactggag cccgccctgt ccccgtccgt atccgagata 11520
ccactgttgc cgccatcata gcctcagctt tcagtgagtg cctgggcctc tgaagccact 11580
gcagatcaga tgaccggcac ctccgcccaa gccccgccct caacgatctg ggaggggccc 11640
tctcacatac tcgtctgtga ttgggccaag tgagtggcac tcatgaccac tccttttcgc 11700
tggttggctg tacgtgtttc cagtcgcgca cgctccttat gagaaaggag gcgggatcga 11760
gcgctacagg ctgctacttg gttcagccag tcgggttgat tctacagcgt ctcatgctgt 11820
atttgccttt ttctgttttt gtccttttgg cttaaaatcg cgatactgga gtctttttcc 11880
taaagataga ccctccctct ggtataaaag agaaaatgca ctccctttga atgaaaaaaa 11940
gggtccacct ccaccacacc atcaggggaa cgccagccag gtgttaaaat gggcaggatg 12000
gaggacagtc tagagaagag aacagatgta gtatcagaac agggggacg agggaaccct 12060
cccttctggc agccgagtct gatggacaac ataagtaaca atgtcagtgt catttggcac 12120
ttaaaaggca ggcaaaatct gtcagagcaa aacactgttt ctttttacta agcatcactt 12180
gccaacagac aagggcaatc ccactgtaaa ttaggggggg actccaaagg gataatcctt 12240
ccttgtgatt tgcactagag gaagaactga cagctagagt cagaaaatgg aagttgccag 12300
cacaaacaca tgtagagact tttctggatt ttgttgtttg gttggttggt tttgttttga 12360
aacagggttg tggtctgaac aaaatcacta tgtaaacctc aaccttgaat caagtgctag 12420
gattacagca gtgagccacc aaacctgcct acacttctgt gccatttttt tgtttttgtt 12480
tttgtttctg gtctttttgg ctgggggtgg ggggagttcg agacagggtt tctctgtgta 12540
gccctggctg tcctggaact cacactgtag accaggctgg cctcaaactc agaaatcctc 12600
ctgcctctgc ctcccaagtg ctgggactaa aggtgtgcgc caccactgct cggattctgt 12660
gccatttttg ctttgttttg gtttgttttt tttttgtttt gttttttgtt tgttttgttt 12720
tttgagacag ggtttttctg tgtagccctg gctgtcctgg aactcacttt ctgtgccatt 12780

tttattagtc ttgttgatta tctttacaaa taagttcaag aaatactaca actaaatctt 12840
taactctcct aacatatatg ctgattaacc taaggtagtg attctcaacc tatgggtcac 12900
gaccccttca gggtcaaacg actctttcac aggagttgca tatcaactgt cataactagc 12960
aaaaattaaa ttaaaataat gtagcaatga taaataattt tatggttggg gtcaacacaa 13020
tctaaggaac tgtattaaag gattgcagca ctgaaaaaca ctgatttagg aggaactgaa 13080
ctactgtcaa gctacaaaga taggtgtatg ataaaacaca tatgctaagt ttctaaaacg 13140
gaatctaggc agtggtgtgt acaaattttt tttatctttt tgctttgtgt tgttggtact 13200
gggaattgag cgtagagtct cttgtgtgcc aatcgtaaat tatataactg agctacttca 13260
ggggttaaga gcatcagctg ctcttccaga gggcctaggt tcaattccca gcacccacat 13320
ggcagctccc aactgttggt aattccagtt ccagaggatt tgacaccctc acacaagtgg 13380
ataaaacacc aaggcacatg aaataaacat ttttttaat tgtggtaaaa caactgaact 13440
actactctaa ctgatgtatg atttttacag tgtatttaca gtgtattgtt taaatatgta 13500
ttgtttaatt agtgtttgac ccaaattcat gccccccaca aagttgtaca tgcatcttaa 13560
tttggaaaca gggtgtttga acatgtaatc agtttataat aaactcacac tgatggttaa 13620
tgtcacaata tggaaggatg agccataacc tgatgactgg tccttataaa gagagaaata 13680
tatttactta ctttttgttt atttgtgatg ggctcacata acctggggtg gcctcaaact 13740
cactacacag gcaataatga cttggaattc ctggtcctca tgtttccacc tcttctaaat 13800
gctacatgcc accacactag gcaagagagg aaaacataga ggcagacagg gaagaaagcc 13860
atataaagaa agactgcagt gatgcagcta catgccaata cacaaagatt tctgacacca 13920
ccaggagagg aaaagcagga accaattctc tcctagttcc ctcagagcaa actcggctcc 13980
atcaaaaccc tgatttgtga cttgtagcct agaaaattat cagaaagaat tctgttttaa 14040
gatgtctagt ttgtggcaat ttgctacagc accctaggga actaatgcac tatttatttg 14100
gcctcacagg aagaacatat aagaagagta tttaagagtg tttgttgggg ggggatgtgt 14160
ttgttagatg aggaaggtgg ctcagtctca cacaacctga tcaatcacag gaactcacgt 14220
ggtagaagac agaacagatt tcttcttaca agttgtcttc tgacctccac acccatgctc 14280
tggcacctgg gagccacccc cccccacaca cacacacaca catagagact aagtaaatat 14340
ttttaaatat ctcctgtcgg gctggagaga tggctcagca gttaatagga ctgactgctc 14400
ttccagaagg tcctgagttc aattcccagc aactacatgg tggcttacaa ccatctgtaa 14460
tgggatacaa tgccctcctc tggtttatct gaaaaatata taatatgata tattaaaata 14520
tgatagtgta ctcacataca ttaaataaac aaatatgaaa aaaaaaacag aaaaacaaaa 14580
agctgggcgt ggtggcgcac gcctttgatc ccagcacttg ggaggcagag gcatgtggat 14640
ttctgagttc aaggccagcc tggtctacag agtgagttcc aggacagcca gggctacaca 14700
aaccctgtct tgaaaaaaaa caaaacaaaa caaaacaaaa aacaaataag tctcctgtca 14760
acaaagatgt tgggaaactg tgtctctctg attgatacag cctttttaat ttggcagcat 14820
ctaaaaacta gaaagaaaaa catgtcagct tctattctag tccaatacat cctggtattt 14880
gccagggagg tatggcagca gcagtggctg ggcaatatcc ttgtccagtt aaacattatt 14940
aaaaatttgc aactataaac gggtttcttt gaatatacat ttgtgcttgg ctgaatattc 15000
ctgtttaacg aaatcataag tatatacctg tttagttaaa agcaataaac ttgtccacgt 15060
gttgtggtat ctgtctgtga cttggaggat aagagatcta aggaagtcaa acccagtttt 15120
tgactactaa ggagttggag gccggcttga actatgtgag aacttctccc aaattttaag 15180
taaataaata aacacataaa cagatacata aacaaacaaa aaagtagcca ggcggtggtg 15240
gtgtatgctt tcaatatcag cattcggaaa ggagaggcag gatttctcag gtcgaggcca 15300
gcctagtcta cagagtgagt tccagggtat ccagggctat acagaggaac cctttccaaa 15360
aaaaaaagaa aaaaagaaaa aagaaaaaga aaaaaagaat agaaaaaaca aaaacaaaaa 15420
caaaacaaaa caaaacccaa aaggcggttg gcccttgagt tgaggcagct gtctatacga 15480
ttgagtttca ggacagttaa gggttagaga aactttgtct tgaaaaacaa atactaatta 15540
atccctaggt taggggaagt ccttctttat tcgagtaatt tttttttcta tttcttgctt 15600
ggttggtttt agggctgggt tttaaaccta ggcactcaag atctcacact taagtaagtg 15660
ctctactaat tgagccacat ctccagccct ttccaagatt ttctttttac atttctgtgt 15720
gttttgtgtg catgcatata taccacatgt gtgcctggtg ctggtgcagg ccagaacaga 15780
gcattgaatc ttccatacct ggagttacag atggttgtat gttgccatgt gggtgaagag 15840
aagcaaactt ggttttctgg aagagtagtc aatgttctaa actgttaaat catctgtcct 15900
acacccccctc ctttcttttt agatttttta aaaaaagttt tatgtttgta agtacacttt 15960
agctgtcttc agacacacca gaaagtggca tcggatccca ttacagatgg ttgcgagcca 16020
tcatgtggtt gctgggaatt gaactcagaa cccctgaaag agaagccagc actttttctt 16080
tttttttttt tttttttttt tttttttttt ttttttttgg tttttggaga cagggtttct 16140
ctgtagagag ccagggtttg gctgtcctgg aactcactgt gtagctggcc tcaaactcag 16200
aaatccacct gcctctgcct ctgcctccca agtgctggga ttaaaggcgt gtgccaccac 16260
tgcccagcgc agctagcact cttaaccgcc agaatcatct ctccagttcc ttaaggtctt 16320
tttttcactc gagggatcaa acccaggact tctcatatgc taggcacaca ctataccaac 16380
aagccctgtg ttgccattgt tgttttgaaa tagttctgct cagtctctgc ttccatagtt 16440
agtctatgtt tgaaagcatg gcatactcag tagtagactg tttacctagt atactggagg 16500
cagtatttta tttttaaaaa gacttatctt tgtgtatgtt tgtgtgtagg tgcccatgga 16560
ggccagagac catatagaat cctctgaact tggaattatt aagtggctga gtcttgacaa 16620
tgatgctagg gatagaaatt aggtcttctg caaaagcaac aggcactcta actgccgaaa 16680
cttttctcca gcccctcacc ctgtgtgtgt gtatatacat atgggcatgg ggtaagacaa 16740
ctttcatcag ttccttctct ccttccacca tgcaggttct atccaactca aattaacagg 16800

ctttgtagtg agtaccttta cttgctggcc ctactttttt gttttgtttt gtttgagaca 16860
ggatttctct gtatatccct actttttttt tttttttga taaatggggt catatagtga 16920
gccttgaaat tactatgtag ccaaagatgg cctctaacct cactttactt tataactgct 16980
gggattatag acttacacca ctgaggggta tatatttgaa caaatgaaat cgtatcttag 17040
aatataaatt taaaactttt gaagaatggg ctggagagat gaattagcgg ttcagagcac 17100
tggctacact tctagaggtc ctgagttcaa ttcccagcaa ccacatggtg gctcacaacc 17160
atctgtaatg ggatcagatg tccttttctg gtgtgtctaa agacagcaac agcatactca 17220
catatataaa ataaataaat ctttaaaaaa ttctcttgaa gaaatgtagc tggcatttct 17280
caaatgaata caaaaataga tacatgaaaa agggatgtta cccttgaatc taagttgtat 17340
tctggctttt acatgtattc tatccccaca aagtgatgtt ttcttcttct aaaaaaaaaa 17400
agtgttttaa aaggccaggt gttgtgccag gcgtggtggt gcacgccttt aatcccagca 17460
ctcgggaagc cgaggcagga ggatttctga gttagaggcc agcctggtct acaaagtgaa 17520
tgccaggagg ccaggcgtgt agtgcacatc tttaatccca gcacctggga ggcagaggca 17580
ggcaaatctc tgcattcaag gccagcctgg tctacagagt gagttctagg gcagcaaagg 17640
ccacacagaa gaatcaggtt ttgaaaatcc aaaaaaaaaa aaaagtttaa agctgggcgt 17700
ggtggcgcac acttttaatc tcagcacttg ggaggcagag gtaggcggat ttctgagttc 17760
aaggccagcc tggtctacaa agtgaattcc aggacagcca aggctataaa gagaaaccct 17820
gtctcaaaaa caaacaaaac aaaacaaaaa aaagtttaaa tttttccttt cttttttcc 17880
accaagacag tcttaccatg tagctctggc tggggtgaaa cttactatgt aaactgggct 17940
ggcctcaaat actgagaccc caagactcat cactatgtac acctaatttt cctccagaca 18000
ggttctcact atgtagttct agctggccta gatctggctg gccctgaact catagagatc 18060
tgcctacctt tgcctcccac acttaacaca aggcaatgtt cctgcttcag cctgcctagt 18120
gctaagataa ctaagatcag gtgtgtacca tcatgtctga cttcacttga ggttttgaag 18180
ttagtttatt actcttgttt tatcaccttt tttttggggg gggggggttc gagacagggt 18240
ttctctttat agtcctggct gtcctggaac tcacttttgt agaccaggct ggcatggaac 18300
tcagaaatcc gcctgcctct gcctcccaag tgctgggatc aaaggtgtgt gccaccaccg 18360
cccagctttg ttttatcacc tttatgaatt ccaccacatt gccatttggc ttacaggtga 18420
tagcaaaaga cttgagtggc cagtgcacag tgagattcca atgtccaact actaaaataa 18480
tttcactgta tccaggcaca tggtcctgtt tcttgttcag caaggtgctt cccatctgca 18540
ttcctacaca gaagacagag gtatggtggc acaacacaaa tgttttatta ctctaaaaac 18600
attaaaaccc agatcccagg atatagagga tgacaaggca aaaaagatta gagaagcagg 18660
taggccctga cactgggggt taatggtcag gtcttggggg tccttcttct gctatataca 18720
agaaacatct agttaagttt agctgtgctt gaatcctggc caataccttc tcttcttgtc 18780
tacaggccac ccaaggacaa accttgtagc agtagctcag gtagaatgac ctggctgaca 18840
gctatccctg atgaggtttg tgagcagtgg gcaagtagta gcccatccaa gggaccaatt 18900
gagtcttctt tctgtaccta tgaccaagaa acctgttctc ctacccaact atttgcttga 18960
ctacctgctc tgttccctcc ctgagaggtg agaagggggc ccctgcctaa gagtgtccct 19020
tccttctcca ccaagtataa gggacagcca aggctcttct tttagctcaa gacccagagg 19080
agtcagatgt tcaagaataa aaaagggtat ccagcaggaa ttgggagcca atctatctga 19140
attaaaagca tcagtccatt tcccagcctc agttttgtac agggaagaat ctcaaaagca 19200
gtaaagagcc aagcaagctt ggtcaggttc ctgtaccacc atttcccaaa tcctgaagga 19260
cccagtgttc ctttggctaa gaactgcagg gcacagactg ggcaggcaaa gcctatcttc 19320
caggctggaa atgattgtgg agctgttctg cctgcatctt cagccgctgg aattcctctt 19380
gaatgaagtc tgtgagggct ttccgcatct ccacctaaga gcaaggatgg tcatggcctg 19440
cattcctcaa ggaccatgtc tggggtaccc tgggagtctg agggactgag aaggccaagg 19500
acagctaggg gaccctggga ggaaactcac agctgacttg ttctctgccc ggagccgctc 19560
aagcacaggg agtgtactga agggcttccc aatcagcacg gtgattttct gtaagaaggc 19620
aggccatcaa gggtgacaag gaacctatag ttccagccca aacaatgcgg ggattcagag 19680
ctatgctctg tacctgatgg gcttgacgtt cctgaaagcc actctccctg ttcaaaacag 19740
gcaattgggc ttgatggtat ccaaggccca gtctagtcaa tatcctatca ataatttgtc 19800
ccactctacc ttgtccctca cccccgcctg atttcaattc tttttctaaa ggtcttgctt 19860
ccagaaaagc cccacttagc tcccctacta catgctcatg ctcagcaaat cttcaaccaa 19920
tcctgtatag tatggacaag gaaacacagg gcagttagca gggaaatagg ttgacaagag 19980
tcagcagtgc ctacccactg tccaaagcgg gggaagtagg gtgggctgtt agggaggaca 20040
tcattcattc ctgtaccaca gtggatagaa agccagccat tgggagaagc agggtaccag 20100
agagaatccc aggtacttct ctcagctacc caacaacctg aaatccccct taacgacatc 20160
taagacagtc ccctccctac ccccaatcca tgactcacca acatgccaca gtggcaggat 20220
gatgggattc agatgacact cagcaatcag ccgtccaatt cctacttcgg agaaatgcca 20280
aagcattggc acaagctggg ccaaacttcc ctgccaacgt ctggccaatg tcacttctac 20340
agtctctcaa tggaacttag tccttgactc accgcctgtc ccttttctac tgctcctgac 20400
acacccacct gagagagaac gaagccaagg acccaagggc catctttgga tgggaagtta 20460
gctgtgtcag gctggccagc aatacttacc ccatttgaag cgcaggaact cagaactcat 20520
gtttactttc ctcctgagag aacacagaga aaaagcttgg gcctagaacc atgaatgctg 20580
gagggccctg atcaagcacc caactgacct tctgggaaga tgtgcaccca gtccccatgg 20640
ttaagcttct ccaaaatgaa gtccatccct ttgtggtgga caccatctcc tgtagagaaa 20700
ggtctgttct ccttaatcca ttctctggag aagaaatcaa actctccaaa ccctagtgaa 20760
gtagattctg gctcactcct ggtctatcca ctttaggatg gaggcagcag ttcatttccc 20820

atagcaccat ccatagacac atcaacatct atttctagag cctgtttctc tttgctaccc 20880
tggcccttca cattttggt ttgaccatat cttcctctta agatggaaat ggtggctcat 20940
gtctgtaatc tcagcatttg ggaagctgag gtaggaagat tgctctgagc ttgaggtcag 21000
gatgggcaac acagtaagat cctgtatcaa aacaaaacaa tattccctca acttcttagt 21060
gcctgtcaaa tgcaaatagc acattctttt tttgtgacag atattactat attagccagg 21120
ttgctcaagt tatagtaatt ctctgttcag cttaatggtg ctgataacag gcatgtatct 21180
gacacttagc tagacagtat ctttcactga aatgatactc atggcaaatc aacctccctc 21240
tgccgtacgg cctgccttgc tggaacctgc ccccctaaca cacacccaat tgctgactac 21300
aaatcagaac cagagattca accttccctc aggtttgtgg gctaagggag aacaattcag 21360
gaagtagaaa acacagaaaa gattggaaaa cttgggaaaa ctgactaata tttcaagaag 21420
tgtgacctaa cacctcttgt gggctagcaa cttggagtca gcttgacgaa caatagtgga 21480
agaataacca cagggtggac agaatcagaa agtggaaggg aagaggctgg aggacaaaga 21540
gcaacactgg ggctatctca ggtcagtgca ttgagaattg cctttcaagg gttaaagaga 21600
tggctcagtg gtcaagaaca ctgaatgctc ttccacaggt cctgagttca attcccagca 21660
accacatggt ggctcacaac catctgtaat aggatctgat gccctcttct ggtgtgtctg 21720
aagaaagcta tagtgtacaa acatatacaa aataaaattt ttatttttta ttttactgaa 21780
gacaggttta ttgggaagct gttctcaggt gagctagttc actagaccca aggaccaaag 21840
ccaggtaagt caccatgtgg gaaggggatg taggggaggg aaagagaaag agagaaatcg 21900
atgtgcttca agaaagaaaa gaaggaaaga gcataaaata aaatcttaaa aaggccgggc 21960
aatagtgata catgccttta atcccagcac ttgggaggca gaggcaggtg gatttctgag 22020
tttaggccac ccctggtcta cagagtgagt tccaggacag ccaggactac acagagaaac 22080
cctgtctcat aaaaccaaag aaaaaaaaag tctcagccca ccctcaacag ggacctgatc 22140
tggcagctca cttagggcca gaagttggga tgatctcagg aaacagagag agccacaggt 22200
gccctacttg ggatggtagt acacagcagg ggtgttcatc acaggtgttt gctcctctag 22260
taaaccccac tgttggaaaa ataacctttg agcttgaaac tgtgaagtaa aagagtgtgt 22320
gtgttttgag acagggtttc tctgagtagc cctggctgcc ctggaactca ctctgtagac 22380
caggctggcc tccaactcag aaatctgcct gcctttgcct cccaagtgct aggattaaag 22440
gcgtgtacca ccactcccag caaggatagg gtatctttgg catacttttt agctactgca 22500
agcacttgta caaaatcact tcagtgctta aaggtgaacc tacaaagtcc taggcttaga 22560
gaaaaatggt ttgtttaaaa acagaaacaa agtatcccaa actctgtttg tttcagtcac 22620
atgtaattag atctttgctt tccttgaaaa taatgttcca tcttctagtt tttgttctac 22680
atttttacaa tgctggggat agtactcagg gctctgtgcc agttaagtaa aaactatacc 22740
actgtgtcac acttccagct ctcagtttct ctgtgtgtat gtgcacacac acaactttaa 22800
tttttttttt ttgagacagg gtcttggttt ctagttggcc tcaaactcat ggtaattcac 22860
caaccccaac ctaacaaacc aaatactggg actacaggca taataactag gttctttctc 22920
tctttcaaga aatctttctt tcttttctaa gacaggattt ttctttgtag tcttggcaat 22980
cttgtaacta gctctgtaga ccatactagc ctcaaactca agagattcac ctgcctctgc 23040
ccaagtgctg ggattaaggt gtgtgcgacc atgaccagct tctatattat tatttttttt 23100
tgataagata gggtatcatg ctaggcttaa tcccagcact cggaaggaag agactggagg 23160
atgagttgcc tcatcgtggt ctcatagagc tagttccagg atagccaaga ctacacagac 23220
aaactgtctt taaaacaaca acaacaataa caaagagaga aagattacca tgtagtccta 23280
tagttctggt cagtctggaa cttgctgtat agaccaggct ggcctcaatc cttatgcccc 23340
tgcttactga gtgatgggat gggattagag gcatatatca ccatgttcat ctcagatcct 23400
tttttctttt taatttttat gttttgcccg catgtatgtc tatgtaaggg tgtaggatcc 23460
cctggaaccg aattacaggc atgagctgcc atgtgactga tgggaattga actctggtcc 23520
tttggaagag caggcagtgc tcttaaccac tgagatagtt ctccaaccac tcagatcggc 23580
ttttaactgc aagaaagtct agaatcagtt caagaagaac agaactatct gtataggcca 23640
taggtacagt atatgcctaa aatatacaag cctctgtgtt ctatccctca aactgaaaac 23700
gaaagaaaaa agaaaaaaat gaaagggctg gagagatggc tcaggagttc agagcactga 23760
acaaaggctc ttccaaagag tctgagtcca atttccagca accacatggt ggctcacagc 23820
catctgtaat gggatctgat ggcctcttct ggtgtgtctg aaaacagggt gctcacacat 23880
aaaattaatc tttttttttt taaagagtga aaacagatgc atacacacag acttttgagt 23940
ttgcagagta aagagcttag agtcaggaca ggtgtaatta tcagaatgct gacatatcag 24000
agcaggatct gagaggaaat ggcactgaga ctatatcatt caacaaaata atatagtctc 24060
ttctggaatg cttagaaagg tggaaataca atcttctacc cttctgacat tctgtaacct 24120
tgtcttctct tatttttaga ccaccctttc tttcattctt tgcttgaaaa ggaatctaca 24180
tacccagatg aaagggcaat gtggatctga atcttaagtt tgccacatgg tagtctatga 24240
ccttggactt ctaagctttg tctagagaat ctgagcccta tctagagaat ggggtcaaga 24300
tggcccctac actaaggggt ttgtgaaagg tactgagtgg tcaatctgag cctcctcatc 24360
ttaccactgt ttgttctccc aaaaacaaaa cccttagcct tctatacatg taccccactg 24420
ccattataaa gtggtaacac cacactgttc tccctgacag gagtggtaga ttcagaaggg 24480
actgcaaata cagcttcctt gaattctcac accagagttt tggactgcag agattctaag 24540
tgatcatccc tcttcctatt ctctgccaac tatactgcca tatcatggct cccctaagtc 24600
cttctaagac agacgcattg accaccagct tccttgtccc caagggaggc ctggacatgc 24660
ctcccagagg agcagctcac ctcgacacac aggcacacat ttgcccaagc tgaagaaatg 24720
ggagtgcagc tccttggtga agcagatgtc tgcagctgct ggggtcctag agcagcaata 24780
gacccaatca tccctgcctc caagacttcc cattgcttgt ccccaactct ccccacccaa 24840

tataatgcct gccatcactg ccatacagaa tgaagctcca agagaaggcc ctaaggagcc 24900
ccacccatgc ccttcctgga aggctggatg ccaactcctg agcacagaaa ggcaaatctg 24960
ggcagaaatg gatgggtcac ttcaaggggc ccattcctcc tcaccaacgc atcaacttca 25020
ggttccagat gtggcggagt tttaggatcc ctgtgaaaaa gtaaagtcct tgatgtgaca 25080
tggctacttc cacactcctt ttaaatttct gatatcctgt ctaaatcaca ttccctggag 25140
gaatcctaag ggggacacac aaaacttcca gcagttaggc agtatatccc ccaaacaagg 25200
taaagttttt caaaagagcc tggtgggaag tcaatctgtc tttggtgggg gcctgttctt 25260
gttttgcctc cctgaaccca gcacactggc accatacccc agagatgagg gtcatccatg 25320
caagactggt ggttggagac ggtgataagg ggggtggcag ggcctcggtt ctcaatgagc 25380
tcatacagca cttccttgtt gtgcacggtg aggtggttca tgtactctgg gaagagaagt 25440
cagtcccaag ctgtaggtca tttccacctg cccgaggctt cttactgtgc cagaaaagcc 25500
cctgccatat tcccagtccg aaaaccccca gccaggccag aggcctgtga acagactctg 25560
ccttggcctt ttgccactca ctggtccaga agcagctgta ggtgccaact aggcccatga 25620
cgacgctgct ggctagagtc caggtgagcc ggggcaccgc ggggaatggc cacttcacat 25680
ggagggcatc tcctccggct ccggccccgc ccccggagcc cagagttcct cttgccccag 25740
gccagccgat gtcctctacc actcaggaga aggatctcca acagactggc agaggactag 25800
actaaggtca cctggtggaa cagctcagca cctccctagc actgttctct ggatagagaa 25860
gagccgctgc tcaggcgacg agacgggact gctgtacaaa gcaatgcgca ctagaaacca 25920
agtcttgacg gcctcactcg acgaatcagc cggaaagcaa ctcgaggaag cgtccttgcc 25980
tgttgtcgca aaacatgcct aggttgcttg acggctgcca gggcgggtct acgccagcct 26040
cgctccggcc cctcccccgt caaggttttg gctccaaacc gccttaagca gtgctgcgtc 26100
tctttgattc cataattcta gtgtctacag gcgacattag ctacacagca ccattacctg 26160
aagctacgca agtcgtaggc tggtggaaac tttttacttg ttctctttcc ctgtcccacc 26220
cgatgtgtat ccggttatgc atgcttctga aggctcacac agagaccgga aaagggcatc 26280
gggtgtccgc gtctattatt ctctgccttt tattgtacta tttctcccag tgatccgggg 26340
gttggggcat ttggttatct ccacgataga gcatttgcct agtattcatg tggtaccaga 26400
actaacaaat ttctgccatg aggcattttt ctaaactgcc actccagtgg ggacctgaac 26460
aggtcagtcg cgctacccat cctgtgcata aggtcctttg ctttgggttt tctgagcagt 26520
cgtcacacag gcaaacttaa tttgggagtg cgcaccaaca ttgtggttca gggcctgagg 26580
accctgagag aagactgcct ctgctctgct ctttgctgca ggggaactgg ccaaagaatg 26640
agcaagccta cctcaccttt ttccctaggt ccaggtaact gttcaatgat agactgctgc 26700
ttcttggaac gggtgatagt aagcatgttt gcgacaacaa gcaaggacgc ttcctcgact 26760
tgctttcagg cggattcgtc gagcagaggc cgccagctcc ccaagttgct attcgcttgg 26820
caaaagaaac acccatcatg ggcgggtggg ccagcaggta gccagttctc aggcacagcc 26880
tactggacag ggatgtggag ctcagccttg cttatggccc gcccctttgg gtgggtctgg 26940
aaaccagctc ctgggctgaa gggtggcgct cagccttgct accttgggac gggaaagttg 27000
tggagccggc ccctttgggt gtgtctggaa acccgcgcct cttcttccca tttccccccc 27060
acacacaccc aactgcgcgt ccttacccgc cttggctgga gctgcgattc tgtggagtgg 27120
aatgttttat tcggttcaac gctgaggcag gcgtggggcc gacttgggct gtggaggtat 27180
gagaatcctg cgggtaggga aagggaggag gttgtctcta ctggggagga acttcagaag 27240
gaaagggaat ttcaagctaa agctgtcttt ggaagactag gaagcattat gctgaggcca 27300
ggaacagtta ttctttctgg ttcatcctca gagtcctagt ggtctcagtc tccaaatata 27360
gcttggccag tgcagaactg ccccagtgct cacttatcca acacagacac cccagtgcaa 27420
ggagaccaaa ataagtatga gtgacagaac aggtgaccag agagaaacaa atatcaaggc 27480
cagctaggaa gatactgtgg cagaaacact aagatgtgca gagtcaggaa gacagaacaa 27540
accagccaga taggaagaaa ctaacagaga ggagaaaggg acagcatgac taagaaaaac 27600
aagcagacag aagcagaggg ccccaggctt caagaatgtg acatgtacag agaacatggt 27660
aaagcgctga gctcaaaaga tatggtaagg cagaggtgag tctgagacaa agataaacct 27720
gagacagatg ctctgagggg ctccaggcca ctcaggaaag ttgaagccag cctgggacta 27780
catagtgagt tccaggccag ccttggttat aaactgagac acacccacgt tttattaata 27840
ttaatatata taaagatata aagatggctg gtgtaatggc ttaacagata aggatattta 27900
ctgccaaaca tgatcgcttg agtttgatcc cttttggtag aaggaaaaaa catgcttgac 27960
ttagacccat gcatatgtgc atacatacac aaacacacac aaatagatgt ggaaaaataa 28020
aacaattaag aaaaataaaa gaaattcctg aagaggttca tgctttagtt tctaatatgg 28080
ataggacaaa ctggaccttc ctgaatctgg gactgtgtat gccctaggct agcaataggt 28140
gctatataga aaatggtatg tagcagtttc tttgtttgtt tgagacaggg tttctccatg 28200
tatccctgac tatcctagaa ctcactctgt agaccagcta gcctggaact cagagatcca 28260
cctgcctctg ccttcaaagc actgggacta aaggtgtgcg ccaccaccca gcagtactca 28320
attaacttaa aagaataagg gttgacctca tccccattgt tagtgaaaat gtaaaatggc 28380
acaaaagcta tggaaaaatt tggtgtacct taaaaaatta aaaataaaat tttcatgaat 28440
tagctgatct gcttcaggaa actaaggtgc tcaaaatttt caaaattgct aaactggagt 28500
gtgattcagt tggtacagtg cttacccaat tatgaggctt acatcgtata gccctcccta 28560
catagtcaat atatgaggcc ctggatttca tctccagcac tgcataaacc aggcttggag 28620
gtatacatct gtcattccag tttgggtgag gtagaggcag gaggatcaga agtttaaggt 28680
cagtttgaca cacacacaca cacacacaca cacacacaca cacacaaatc actacagggt 28740
tacagttata gcttagaggt agagcacatc tttagcatat ataaggcccc atcttcaatc 28800
cctagtacca cacattaaaa ataaagtaat taaataaata aatacaagaa ttcaagtagt 28860

gggtgtaccc caatgctgat ggacaaagtg tttctgagta tacactggaa tattaatcat 28920
ccttaagaag gaagaagaca gccgggcatg atggtgcacg ccttttatcc cagcacttgg 28980
gagacagagg cacatgggtc tctgagttca aggccagaat ggtcatagag tgagttcagg 29040
acagctgggg gatacacaga gaaacctgtc ttggagaaaa aaagaagaag aagaagaaga 29100
gaggctggag agggcactca gcagtgttta ccactcttgt acaggatctg agtttggttc 29160
ctagtgcctg cattgggttg ctcacaactt ctaactgcag cttaagaggg atctcatatg 29220
tctggtcctt gagggtacct tcactcatat gaacataccc acttacagac atatacataa 29280
ctgaatgtga aatacatctt tttaaataat ggaaaagaat ttctgaactg acaagatatg 29340
tcagtgggcc tgcgtggcct ccttgggcat ctgcatgtgc atatacctcc acatacatac 29400
acataattaa aaataaaacc atccgtaatg agatctgact ctctcttctg gagtgtctga 29460
ggacagctac agtgtactta catataacta aataaataaa taaataaata aataaataaa 29520
tatctttaaa aaaaacctaa gagagattaa ggctttttaa aaaagaagat aattttgaca 29580
atggttatac aacaagaata aaccatgagg acatggtatt aaataataag acagtctcac 29640
aaagacaact atgttgttct actttagtga tgccattagt gttgacaaat gtagaagcag 29700
aggtacaatg gtgcttgtag ggtttttttc ctgagtatca caaaagttta cttagcaaga 29760
agttcatata acaatgtgta tgacctggct ctttctgaac tcttctactg aagaactatg 29820
gttttgatgc tttacactga gagatcccct cataatttcc ccaaagcgta accatgtgtg 29880
aataaatttt gagctagtag ggttgcagcc acagtaagtc ttcccttgtt attgtgtagc 29940
cagaatgccg caaaacttcc atgcctaagc gaactgttga gagtacgttt cgatttctga 30000
ctgtgttagc ctggaagtgc ttgtcccaac cttgtttctg agcatgaacg cccgcaagcc 30060
aacatgttag ttgaagcatc agggcgatta gcagcatgat atcaaaacgc tctgagctgc 30120
tcgttcggct atggcgtagg cctagtccgt aggcaggact tttcaagtct cggaaggttt 30180
cttcaatctg cattcgcttc gaatagatat taacaagttg tttgggtgtt cgaatttcaa 30240
caggtaagtt agttgctaga atccatggct cctttgccga cgctgagtag attttaggtg 30300
aagggtgggt gacaatgagt ccgtgtcgag cgctgatttt ttcggccttt agagcgagat 30360
ttatacaata gaatttggca tgagattgga ttgcttttag tcagcctctt atagcctaaa 30420
gtctttgagt gactagatga catatcatgt aagttgctga taggtttcca gttttccgct 30480
cctaggtctg catattgtac ttttcctctt actcgactta accagtacca acccagcttc 30540
tcaacggatt ttataccatg gcactttaaa gccagcatca ctgacaatga gcggtgtggt 30600
gttactcggt agaatgctcg caaggtcggc tagaaattgg tcatgagctt tctttgaaca 30660
ttgctctgaa agcgggaacg ctttctcata aagagtaaca gaacgaccgt gtagtgcgac 30720
tgaagctcgc aataccataa gccgtttttg ctcacggata tcagaccagt caacaagtac 30780
aatgggcatc gtattgcccg aacagataaa gctagcatgc caacggtata cagcgagtcg 30840
ctctttgtgg aggtgacgat tacctaacaa tcggtcgatt cgtttgatgt tatgttttgt 30900
tctcgctttg gttggcaggt tacggccaag ttcggtaaga gtgagagttt tacagtcaag 30960
taaggcgtgg caagccaacg ttaagctgtt gagtcgtttt aagtgtaatt cggggcagaa 31020
ttggtaaaga gagtcgtgta aaatatcgag ttcgcacatt ttgttgtctg attattgatt 31080
tttggcgaaa ccatttgatc atatgacaag atgttgtatc taccttaact taatgatttt 31140
gataaaaatc attaggggat tcatcagtgc tttacaaggc ttctaacatg atgatgctat 31200
ttcctgttat ccatccaata ttattctgct gcccactaat tgccatcttc acatatttgt 31260
cagtcacaag attcataaag ggatcaaagc cctgcaatat tccttgtata tatctgcctc 31320
catttaactt caatgataac ttattttcca caaatgtctt cagttctagc gagtgggcct 31380
tgcacatggt cactgtgctt gttgtgtttt gaagcagggt atcacgatat aactgaagaa 31440
tacctcaacc ttaggatctc attgccttca cctcccatat gctggaatta cagaagtatg 31500
cctccacacc taggcttgtt tttggttttt gaaaagacag tctcacgact ggggagtagc 31560
tagcggaaga gtgcttctct agcataccta caaagataac gactgtaagt tcaagcctca 31620
gcagtgaaaa ggagaaaggg agggagggag ggacggaggg agggagggag ggagaaagat 31680
tgatattggc tctgtgggtt tggtggcata tacctctaat cccagtactt ggatggtgga 31740
aggaatctca agggttcctg aggtcatcct aagctacatg gacaagtttg agaccagact 31800
gggatacatc agtccttgtc tcaaaagggg aagtataagt aaaaaacaca gtaaagaatg 31860
agtcatccag gcttggtgat tatgaactcg tgatcctatt actcaggagg ctgacaggag 31920
gattgctcca agtttgggac ctgtgcttta ctacagagca agactgtctt tcaaaaactc 31980
acgcagaggt cctgggggga agtcagtaaa aaacgtgcac ataaaaaaac aggcgccggg 32040
gtggtggcgc atgcctttaa tcccagcact cgggaggcag aggcaggcgg atttctgagt 32100
ttgaggccag cctggtctac agagtgagtt ccaggacagc cagggctata aagagaaacc 32160
ctgtctcaaa aaaccaaaaa aaaaaaaaaa aaacaaaaac aaaaaaaacc aaaaaccaaa 32220
aaacaaaaac aaaaaaaaaa cccaggcatg gtaaaatgtt cttgtaatta cagcactagg 32280
gaggcagcga caattggacc cctacggctc aatagctagc caaatttcat gagttccaat 32340
ccagtgacac caaccagggt ggatggctcc tggggttgac ctctgacctc caaatgtgca 32400
cacacataaa tgcatataca ctcacaagca cccatatgct tatacaatgc atacatatat 32460
acattgaaaa ataggctgaa aggtaaaaaa cctagacaga ttttgttgct ggagctgacc 32520
ccatgtgaac tcctggccat aatcttggca aagaaggtat actcactgag tacacaattt 32580
cacctgggga agatgaaaaa gctctgccga tctaggatgg taacatttgt gcttctggct 32640
attgagttct taaaagtctg agatggtttt atgccacacc acatgtattt tattaggtgg 32700
tcaagctctg tgtacagaag ttgaaatacg ttccaggaag agattcaaga aatgttcagg 32760
gccaacagca gggagaatac taattagctg agatcagctg cagtgccaat gccctttgga 32820
caacctggat tcctttggag ggttcctgat gcacacatag caatgcgtgg ccttgttatg 32880

gctcccctgc tcattctcct ggttggtggg actgaagcct ttcgtatctg tgccttcaat 32940
gcccacagac tgacactggc caagctaacc aaggagtcag tgatggatac cttagttcag 33000
gtaagttcat tgttgcagaa atttgtgtcc ataggacctt gactaaaaac tccccatcct 33060
gcctgaccat aaaggagggg acatctggaa aagtgaatgt acagatgtga agagtctttc 33120
aaccatcatt taattaccaa cttcctcctc taaaaatggt aaggtataat gaatgatgtt 33180
gtttgtttcc tgagtttctc cttgtctcac tgctgtctgc agccactctg gtaccaatct 33240
ttacataaag ctcctccatg ttgacagctc tgaattgagc tggtatccct cattccctgc 33300
agatcctagc ccgatgtgat atcatggtgc ttcaggaggt agtagattct tcccagaata 33360
ctgtcccctt tctgcttcaa aaacttaaaa ggtaaagaag gaccctttca atgtaatctg 33420
cattccacct agagccttaa gagccctaac tctggttttg tttcctggta tatttgacag 33480
ttccaggagt tacagcttcc taaacagctc attgctgggg cgcagcacat acaaggaaaa 33540
gtatgtgaat atctacaggt gagcacaggg gtcaagaatc ctcatcatga caattctagg 33600
taggggtctg aagacccagc tattgtattg tgttcaagaa gataaatcag gtagatgaat 33660
tgctggagcc catgagttca agaccagttg ggttataatg aaaatggatc tcaaaaaaaa 33720
caaaacaaca acaacaaaaa aaaaccaacc tcataaaagt tctctggaga gatggctcag 33780
tagttaagag aacttgttgt tcttccagag gactaaggtt ttattcccag aacccacaca 33840
agagcacaca accatcacta attccagttc caggagatct ggcatcctct tctggcctct 33900
atagacccat gacgctcaga aatacatgta ggcaaaacac tcataaaaat aaaataaaaa 33960
taaatctcta aaccaggagg aagtggtggc acacaccttt aatcccagca ctcaagaggc 34020
atgggcaggc agatctttgg ccagactagt ctacaaatca agttccagga cagccaggat 34080
tctcttatgc agagaaaccc tgcttaaaaa atagaacaaa cagccgggca tggtggcgca 34140
cgcctttgat cccagcactt gggaggcaga ggcaggcaga tttttttaa aagatttatt 34200
tatttattta tttatttatt tatttattta tttatttatt tatttattaa atgtaagtac 34260
attgtagctg tcttcagaca ctccagaaga gggggtcaga tcttgttatg gatggttgtg 34320
agccaccatg tggttgctgg gatttgaact ctggaccttc ggaagagcag tcgggtgccc 34380
ttaccaatga gccatctctc cagcctggca ggtagatttc tgagttcgag gccagcctgg 34440
tctacagagt gagttccagg actacacaag aaactctgtc ttgaaaaacc aaatataaat 34500
ataaataaat aaataaataa ataaataaat aaaacaaagc caggcaatca tggcacacgc 34560
ctttaattcc agcacttggg aggcagaggc aagtggattt ctgagtttca aaccagcatg 34620
gtctataagt gagttccagg acagccaggg ctacacagaa aaaccctgtc tcaaaataac 34680
caaaattaat taattaatta attaattaat acaaaataaa gcagaacaca agacacagcc 34740
acagaatcaa ctaagcacgg ctcatacaga atcacagaga ctgaagtgat aatcacagac 34800
cgtgtatggg tctgagctat gttcatggtt gtgcagcttt gggactccta attccttttg 34860
cccctttcct acaactgggt tgcctagtca accttgatat gagggtttgt gcctagttgt 34920
aaattgttat accgtgtttg gttgatattc ctgggagcct gttctttttg tttttgaagg 34980
gaaatggagg aggagtggat gggggcaggc gagaaactga gtcgtatgta atgtatgaga 35040
gaataaaaca aagatcctaa attaaaaaac aacaaccaaa acagttccag ggagctggag 35100
gcataagtcc tgatcactga gtcaagatga tccccagtgt aaattgaaaa actccaagcc 35160
aggagtggta tcccatgcct ttaatcccag cactcagagg cagagacaga tggatctctg 35220
tgagtttgaa gtcagcatag tttacagagc aagttccagg ccagccaggg gtacacaaaa 35280
agaaacccta tcagagatga gagagagaga gagagaaaga gagagagaga cgagagagag 35340
agagagagag agagagagag agagagagag agatccaaga cagacagata gggttacata 35400
atagaacttt tcttttaaaa aaaaatagaa ggaataagaa aaaaccctaa gttctagaaa 35460
aaaatggttg tacccccatg agagtggcag aacaaggcgc caggtatctt cctctttgag 35520
cactaactct actcttttta tataagattt ggggccacta cttcctacct cttggtcttc 35580
ccaggtctga caagacacag gttctaaatt tctaccaata caacgacaca gatgatattt 35640
ttgcccgaga accatttgtg gcccatttca ctctccctag caaaagtaag aggggtaaaa 35700
aaatctgggc ctggtacaag gaaataggag ggggatgtgg ctggttttcc tgtgggttgg 35760
tttctggggg aagcagaggt ggttatgtag aaggctggca gctgtggcag cctcagcttc 35820
ctggaccttg cagctcttcc aagtgtggtg ctggttcctc tccacaccac tcccaaggat 35880
gttgagaagg agctgaatgc cctctatgat gtgtttctgg atgtctacca acgctggcag 35940
aatgaggtag ggtgatagaa ttccatggca gaggggatgg tgcaaatgcc ttttagcaag 36000
acgcagtggc ttctgtcgtc tctagaatgt gattctgctt ggagacttca atgcagactg 36060
tgcatcgctg acaaaaaaac gtctgaaaag cctgctactc cggactaagg caggcttcca 36120
ctgggtgatt cctgatgggg aggatactac agtgcgggcc agtactaact gtacctatga 36180
ccgaattgtg gtgcatggac agggctgcca aatgctgctg aaggctgcag ctacctttga 36240
ctttcccaag aggtttcagc tgactgagga agaggtagag aagctgggtg ggcaaaatgg 36300
ctgagggcag aattgtggag gtgagccaga tactgacact tcattttgtt ctctcctcag 36360
gctctccgca tcagtgacca ttatcctgta gaagtggaac tgagccaggc cactccactg 36420
agtatcccac cccactactt agccgctcta ctgctgtcac tcctgccatc tcagctggac 36480
tagatagcca ttgacttgac cagggcactg gtagtctcag atgtgaaagt caatgacagc 36540
ttttttaaat agctgcttgc tattctgaga aagctagggc tgggagtata actcagcaac 36600
aagtgccttg ggcttgaatc ctgttgggta gacaggactt cacctgaggt agttaggaac 36660
aaagctccag tacaaatggg ctcagatcat acctaagatt tctgcaactg atacaaatga 36720
gctcatactg gaaaaacctt aggtctaagt aatcttgctt ctccatgcac tatactacat 36780
ggctctgcca gtgttgtcag gtttaaaata ttcaataaaa tcattttaac cttttgcatg 36840
tatgcttaat tcaataagct gtcccaattg accaaattta aaacctcatg gtatttaaga 36900

atcagaaaca aaaacataag cgaacttgat gcgagatttt attattgaac gatttggtag 36960
ggtataggag aactattgaa gccttcagga atgcagggct ctccacttgt ccagaggacc 37020
acgattgggg atatatttga caccacagcc atcaggaatg agccgcttct cagcaaccat 37080
gtcttcaaat tcatctgcat taaacttggt gaagccccac ttctttgaga tgtggatcta 37140
gaaggaaata gaaaataaca aactcagcaa ggaggaccag agcaagaaaa cagaagaact 37200
taaataccac tacctacctt ctggcggcca gggaacttga acttggctct tcgcagagcc 37260
tcaatcacat gttccttgtt ctgcaacttg gttcggatgg acatgatgac ctggccaatg 37320
tgaaccctgg ccactgtgcc ctggggcttc ccaaaggcac cacgcatccc tgtctggagc 37380
ctagatcagg gacaacattg tgatcagagg cctgattacc aactgccaga aaattgtctc 37440
caaggtccct tgaggcagcc catatagata ataggctaca tacactacca aggaggctgc 37500
tgtttgcaac cattgcacac caaagtcccc atgggaaaag gggacttggt cggcttaatt 37560
agctgcacaa aaaatcaaac aaatccccaa aaatactgct ggacagtggt ggtttttaat 37620
cacttgggag gcagagcagg cagatctcag atatgcctgg tgtacattaa gttcccggac 37680
agccagggat acagagaaac cctgtctcaa aaccaaataa aacaccagct tacctgtcag 37740
ccccagcaca ggacaacatc ttgttgatac ggatgacttg gaaaggttgg agcctcactc 37800
ggatatgaaa gccatccttg ccacaactct ttaccatgta tttgttggca caaatacggg 37860
cagcctccag tgatgaatgg ggaaataaca gcagtgaaac agggtgtcag gactttgaat 37920
aaattataat atataatcca cacaagtcca gaatcaatta cacacgggca tgtgtccatc 37980
ttatccagca ctggagtgat ttctgagttg agccagctgt ctacaaagtg agttccagga 38040
cagccaaggc tacagagaaa ccgtctcgaa aacccaaaaa aaaaaaaaaa aagagccaat 38100
taacaaatgg gagctgtctc accttcagaa gagagttgct catattcatc tgacaccata 38160
tggccacaaa gtgggaattc atcaactttt gctttcttcc gtcccaaatc aaagattcgg 38220
atcttagcat ctgaaaaaca cgtggtttaa agttctcgag aagtggggaa tagacaaaca 38280
ccgatccagt tcgaagtttg tcactactta ccagggacac cacggcagaa acgagacttt 38340
gggtatggct tgttcttaca ataccggtaa ctatagaagg aaggaaactc aggtgtgatt 38400
aatggtgaca acaggactaa accagctctg aatccagttt ccattcaggg taactgattc 38460
taaggcaact tgttgttaaa caattacaca aattataaaa cagaaaaaga atcatgcact 38520
agcatcaccc ttctaaccag agttgggtga gatatcacaa ttttcttagg catgtgacag 38580
aagacccgct ttagccacgt taagagaata tggcgtcctc ttcaggacac gcgtttaggc 38640
ggcgctacgg actgtcagcc cttggtgtcc tgttaaaaac cacaaagctc aaggccggaa 38700
ataaaagacg gacgcaaaag tgaccaagaa ggcctggtag gcgttgtaaa aaggctcatt 38760
tgcgacgcag aggattggat gcacacggac atgctagttc tacccaagcg ctttagtatt 38820
gggtaacgcc acccacagcc tcaaaataaa ggtcccagta aagtattaaa gaaaacccga 38880
agtcagtctt tacttcaggg tctctaagtg gcagagagtc cacccataaa aaaaaagtct 38940
ttcgaaagaa atgcattttt ccctcagttt tcacagtagg aagattgcat gggttcagca 39000
ctcaccaccg ggcggggcgg cggcccatga tgacaatagg atcgtcggtg gctgtaatga 39060
aacagtcaaa gaatacagac acagaataag tgtaaaaaaa atcccaaaga aaaattacga 39120
gaccatcgta aaaaaccgtg accgaactca ctcaccgcgc cagaggaaaa gagacgctcc 39180
cacgcctgct cacgccttat ataggcggcc tatcttccac tgtaacaacc atagagttac 39240
agccaggggg tggtgtcgac gctctatcag acgctaagat catagatgca gcgctgctct 39300
gcgcatgtgt accaggcggg accgaacgaa gcacagagct tattggtgac tggataccgg 39360
aatagggaac tgcatgctgg cgaatgggat ttcgacacat aggcaagaga aaccagcttc 39420
cgcctggcca tattgggcgg agagccattg acaggttttg ctattacgta tgattgccaa 39480
tattctaaga cttgaaaagg ggagtctttc tcagtggaac tggaccgtca cggaggtgtt 39540
cctttgctta cagggctcta gagtgctggt tcaggccttc ctcaaggcta caggaaatgg 39600
cgcgaacgtt ccatggtagg gactccgaag ttcctttgtc acccgggagg ttggagaccc 39660
acccacaaag ctgacgtcta agagccaaca cagagcacca tgataggtgc ttgattgctt 39720
tcaggagacc aattaaaatc tcccttagag gaaaaaaatc tccgttaggc gctcactgtc 39780
ctcctcgcta gttgggcccg ccccactcta attctcagga aaagacgtgt tcttagggag 39840
actggagact gggcaaagac gatggcgata tcattacttc ctactagccc caggagggtc 39900
gcggtgctgt gggccacgcc ctagtaggag agtcggagtt cctttgcccc agattccgag 39960
agaaaaacta ggtgtagcac acctcttctt ttcactatga agaattcaaa atgtacatct 40020
gggtgtgtct agaaggattg gcagctaacc actaactact ctcccatata aggttacaat 40080
ctagggaggc accagaacgg gaacactgtt ctgatc 40116

## Beschreibung

Die vorliegende Erfindung betrifft eine DNA-Sequenz, die murine DNase X sowie die dazugehörigen Kontrollelemente kodiert, das murine DNase X-Protein sowie anti-DNase X-Antikörper. Die vorliegende Erfindung betrifft auch die vorstehenden Verbindungen enthaltende Arzneimittel, die vorzugsweise zur Prävention und/oder Behandlung von systemischem Lupus Erythematosus (SLE) oder anderen Erkrankungen, bei denen Apoptose eine Rolle spielt, eingesetzt werden, sowie auf diesen Verbindungen basierende Diagnoseverfahren und Kits. Schließlich betrifft die vorliegende Erfindung auch ein nicht-menschliches Säugetier, bei dem das DNase X-Gen verändert, vorzugsweise inaktiviert, ist. Das erfindungsgemäße nicht-menschliche Säugetier eignet sich zur weiteren Erforschung von Erkrankungen, bei denen Apoptose eine Rolle spielt bzw. zur Entwicklung weiterer Therapieansätze.

Anders als beim pathologischen Tod von Zellen (Nekrose) handelt es sich bei der Apoptose um ein genetisches Programm, das zum Zelltod führt. Bestimmte externe oder interne Faktoren können diesen programmierten Zelltod auslösen. Apoptose ist ein essentieller Prozeß der notwendig ist, um bestimmte unerwünschte oder schädliche Zellen zu entfernen. Dieser Prozeß ist unter anderem von entscheidender Wichtigkeit bei der Embryonalentwicklung, beim Aufbau und Erhalt des Nervensystems, bei der Entwicklung und Aufrechterhaltung von Geweben mit hoher Teilungsrate, wie z.B. der Epidermis und epithelialen Zellen (z.B. des Verdauungstrakts). Desweiteren spielt die Apoptose eine wichtige Rolle beim programmierten Tod von Zellen in physiologischen Prozessen, wie Zellen des Nervensystems durch Entzug von neurotropen Faktoren, bei der Atrophie von Prostata, die durch einen Androgenentzug (z.B. bei Kastration) ausgelöst wird und dem Tod von Tumorzellen durch cytotoxische T-Lymphozyten. Desweiteren spielt die Apoptose eine wichtige Rolle bei pathologischen Phänomenen, wie dem Tod von Thymozyten nach Bestrahlung, dem viralen Tod von Zellen nach einer Infektion mit AIDS oder Influenzavirus, dem Tod von Krebszellen in malignen Geweben, dem Arzneimittel- oder Chemikalien-induzierten Tod von Zellen nach Antitumor-Medikamenten oder bakteriellen Toxinen und dem Tod von Tumorzellen durch eine Thermotherapie. Die Erforschung und das Verständnis von molekularen Mechanismen der Apoptose ist ein wichtiger Schritt zum Verständnis der Bedeutung und der Rolle des programmierten Zelltods in der Entwicklung von vielzelligen Organismen und der Unterdrückung und Bekämpfung von Krebs. Beim gezielten Absterben der Zelle wird die DNA der Zelle normalerweise abgebaut. Ein charakteristisches Phänomen, das normalerweise während der Apoptose auftritt, ist die Kondensation des Chromatins und die Fragmentierung der Chromatin-DNA. Insbesondere die Fragmentierung von Chromatin-DNA in nucleosomale Einheiten ist eine charakteristische Änderung, die bei apoptotischen Zellen auftritt, unabhängig von der Unterschiedlichkeit der auslösenden Faktoren. Es wird angenommen, daß DNasen diesen Abbau vornehmen. Es kann aber bei diesem Abbauprozeß Störungen geben. Einmal kann der geregelte Eintritt des Zelltodes als solches nicht richtig funktionieren, was eine Entartung der Zellen und damit ein Tumorgeschehen fördert. Andererseits kann aber auch die DNA nicht vollständig abgebaut werden, wobei die übriggebliebene DNA als Immunogen wirkt und Autoimmunkrankheiten, z.B. systemischen Lupus Erythematosus, auslösen kann. Die übriggebliebene DNA kann auch als Schleim in der Lunge auftreten, was bei Patienten mit dem Krankheitsbild einer zystischen Fibrose auftritt. Systemischer Lupus Erythematosus (SLE) ist eine multifaktorielle Autoimmunerkrankung, an der z.B. alleine in den USA über eine Million Menschen erkrankt sind. Spezifisch für SLE ist das Vorhandensein von Anti-Kern-Antikörpern, die gegen "nackte" DNA sowie gegen Nukleosomen gerichtet sind. Es wird davon ausgegangen, daß die resultierenden Immunkomplexe akkumulieren und eine Hypersensitivitätsreaktion des Typs III auslösen. Diese manifestiert sich dann als Glomerulonephritis, Arthritis und allgemeine Vasculitis. Obwohl die Ätiologie von SLE bisher unbekannt ist, so deutet doch vieles darauf hin, daß eine verstärkte Freisetzung oder ein gestörter Abbau von DNA-Protein-Komplexen nach dem Zelltod die Krankheit auslösen oder zumindest verstärken können. Es konnte gezeigt werden, daß allgemein die DNase-Aktivität im Serum von SLE-Patienten niedriger ist als bei gesunden Menschen. Bei der Bestimmung der DNase-Aktivität im Serum der Patienten konnte jedoch bisher nicht zwischen den vier humanen DNase-Proteinen aus der DNase 1-Familie (DNase 1, DNase X, DNase 3 und DNase 4) unterschieden werden.

Somit liegt der Erfindung im wesentlichen das technische Problem zugrunde, Mittel bereitzustellen, die eine Prävention oder Therapie von SLE ermöglichen. Desweiteren soll untersucht werden, wie die Aktivierung von DNasen, insbesondere der DNase X, genutzt werden kann, um unerwünschte Zellen oder entartete Zellen abzutöten und zu entfernen. Hierzu zählen insbesondere Tumorzellen. Außerdem geht es darum, wie DNasen zur Prävention oder Therapie von zystischer Fibrose eingesetzt werden können.

Die Lösung dieses technischen Problems wurde durch die Bereitstellung der in den Patentansprüchen gekennzeichneten Ausführungsformen erreicht.

Die vorliegende Erfindung beschreibt eine bisher unbekannte DNase, die eine DNA-Fragmentation katalysiert. Hierbei wird Chromatin-DNA in Mono- oder Oligonucleosomen-Einheiten gespalten. Dies ist auch ein charakteristisches Phänomen der Apoptose.
Es konnte im Rahmen der zu der vorliegenden Erfindung führenden Untersuchungen gezeigt werden, daß DNAse X eine wichtige Rolle bei der Apoptose spielt und in Zellen mit hohem Turnover exprimiert wird, man somit davon ausgehen kann, daß ein Ausfall der DNase X-Aktivität bzw. der zu geringen Expression des entsprechenden Gens mit verschiedensten Erkrankungen in Zusammenhang steht, bei denen eine gestörte Apoptose eine Rolle spielt. Diese sind beispielsweise Krebs, AIDS, SLE oder zystische Fibrose. Daher sollte eine Prävention und/oder Therapie durch die Verabreichung von DNase X (als Protein bzw. über eine Gentherapie) möglich sein. Schließlich wurde auch ein Tiermodell entwickelt (DNase X-Knock-out-Maus), das z.B. ein weiteres Studium der Bedeutung der DNase X erlaubt.

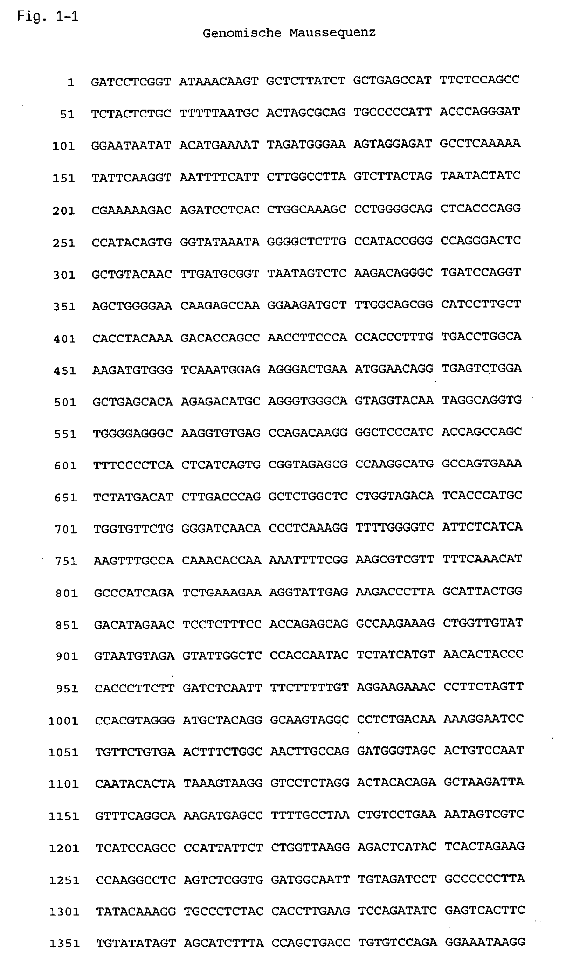

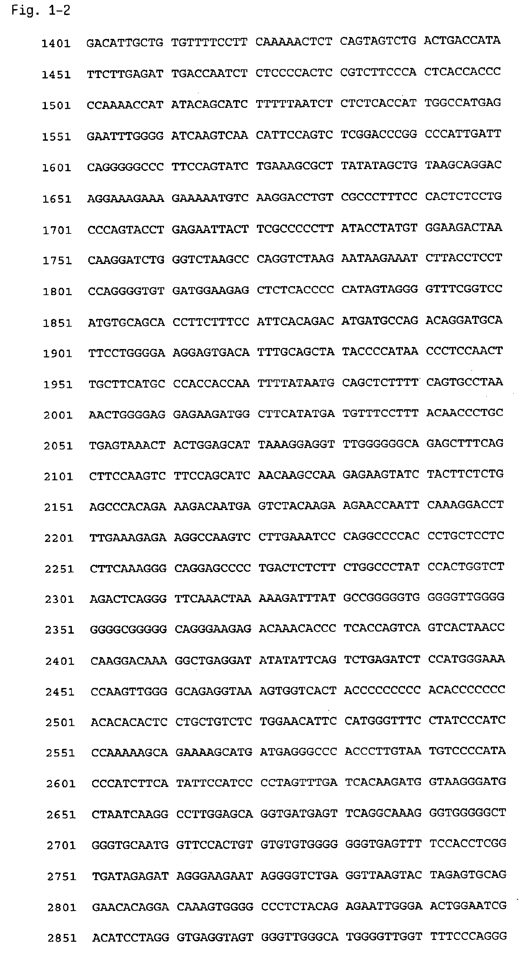

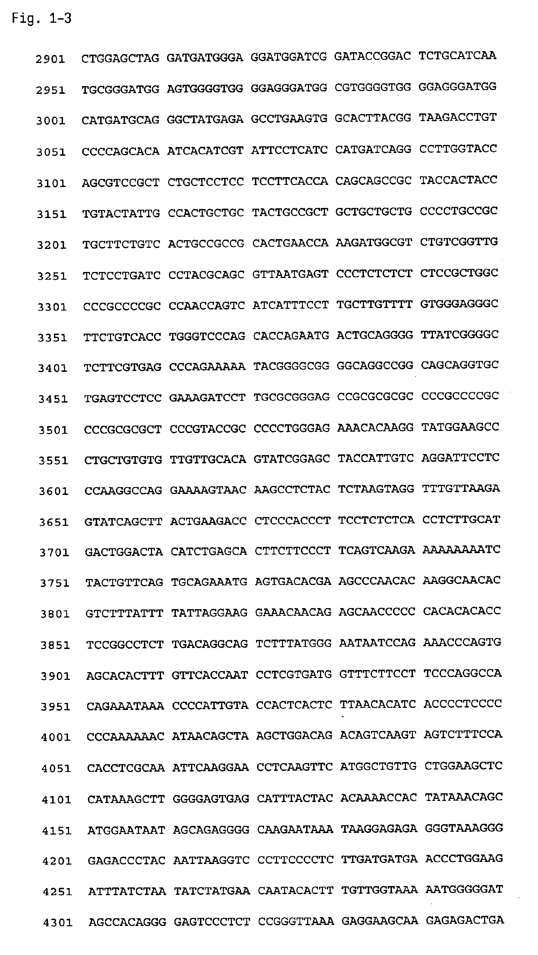

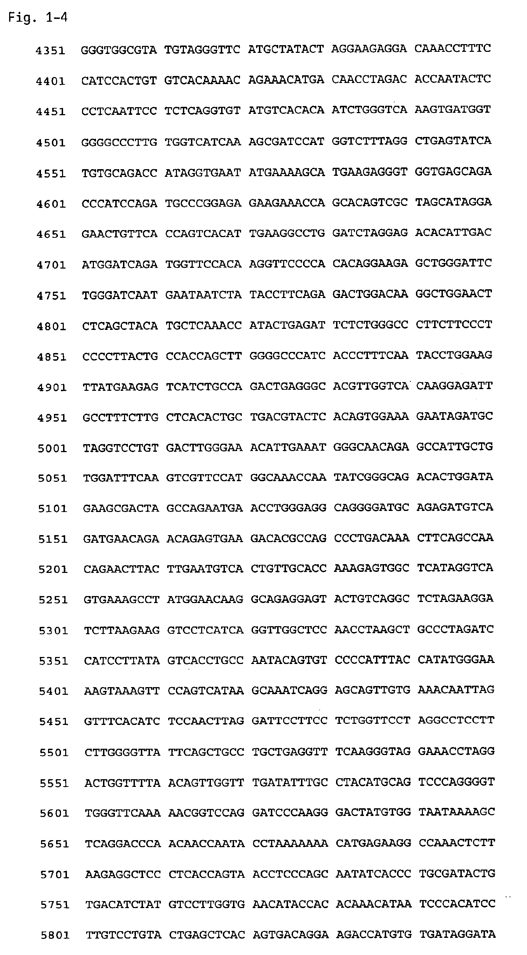

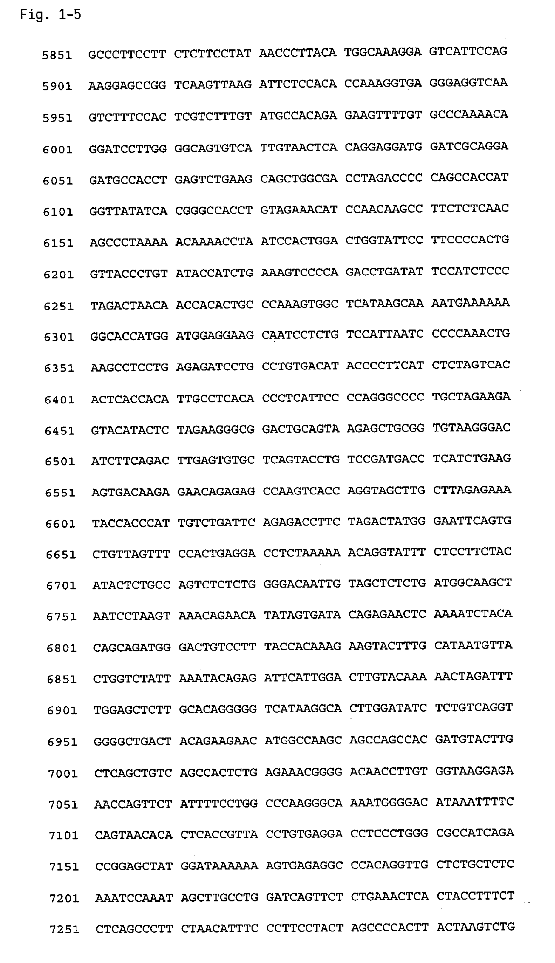

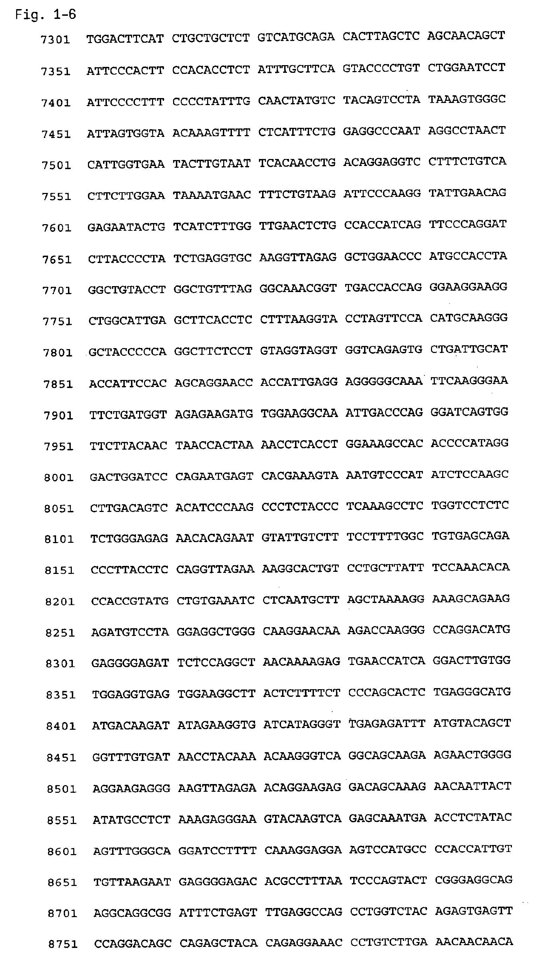

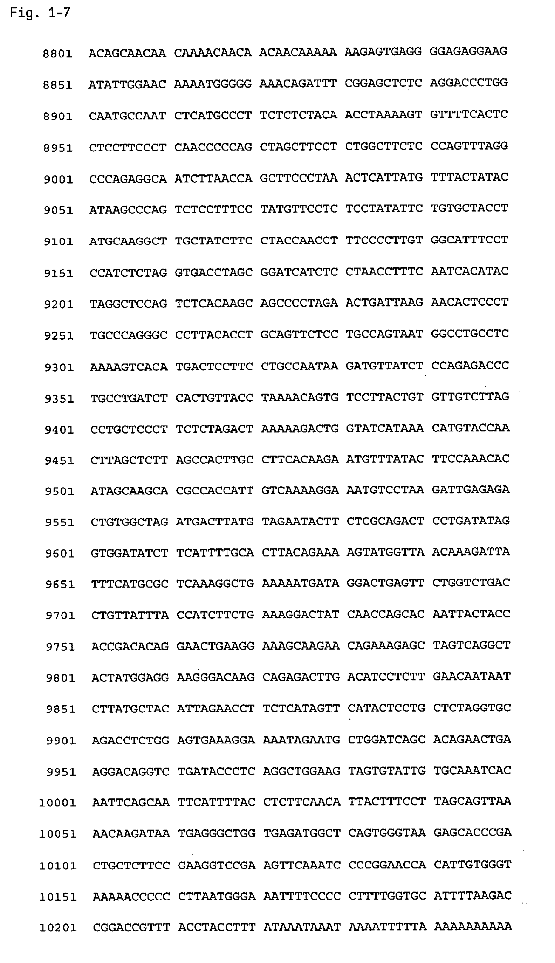

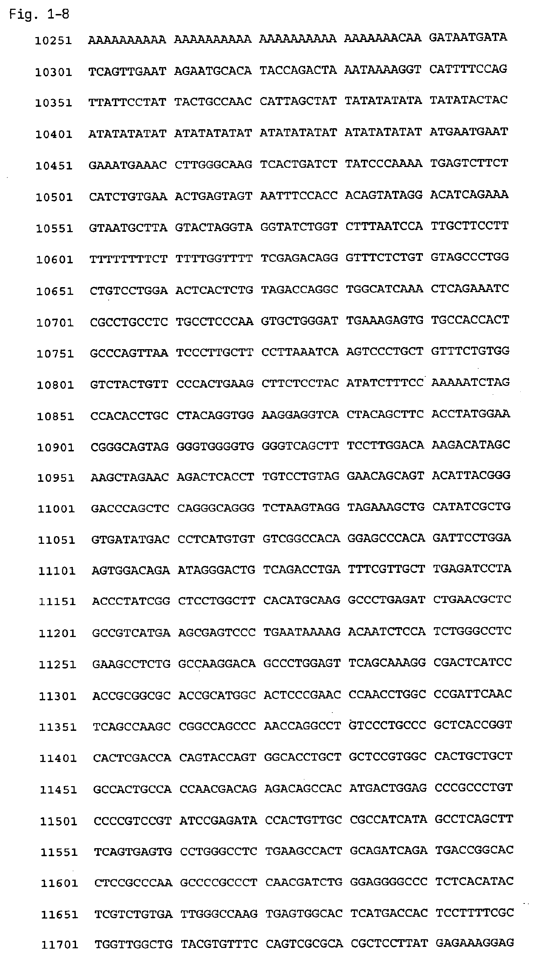

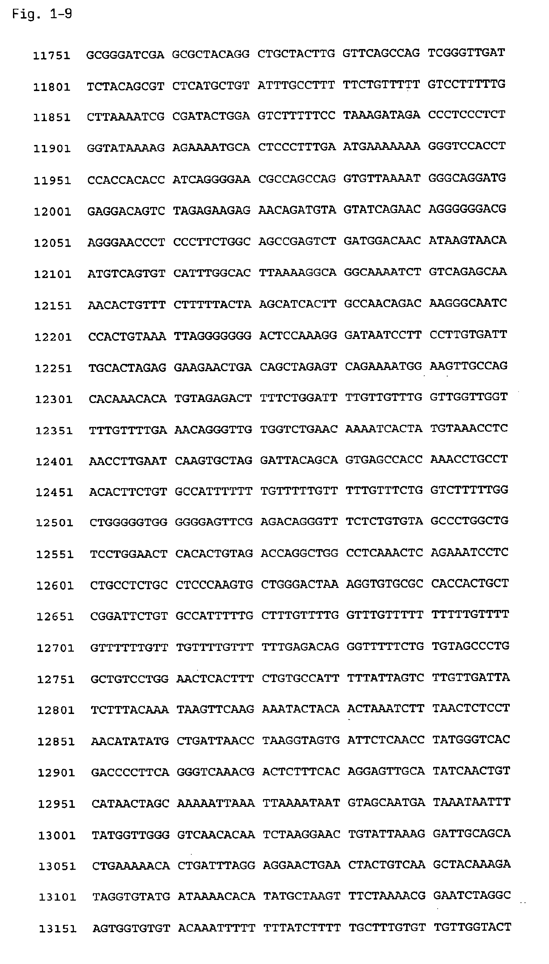

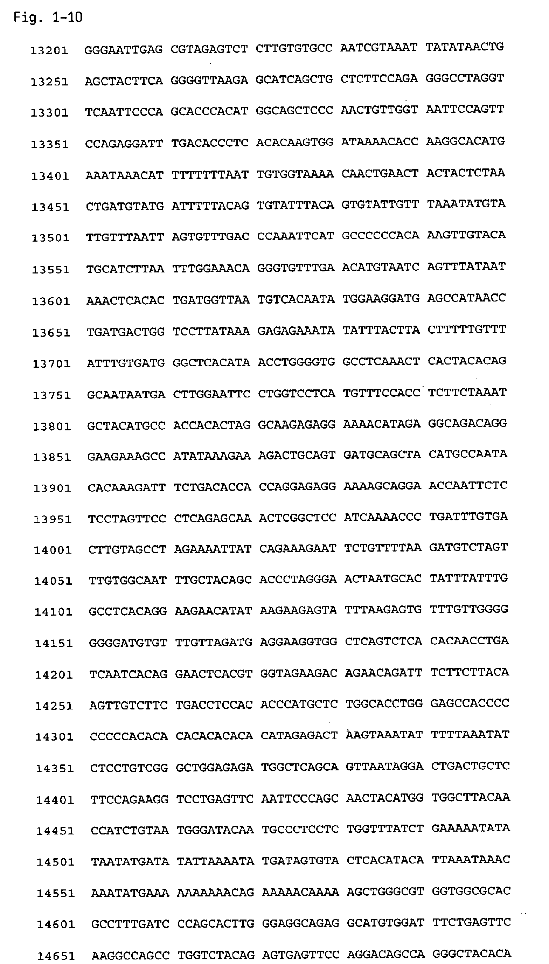

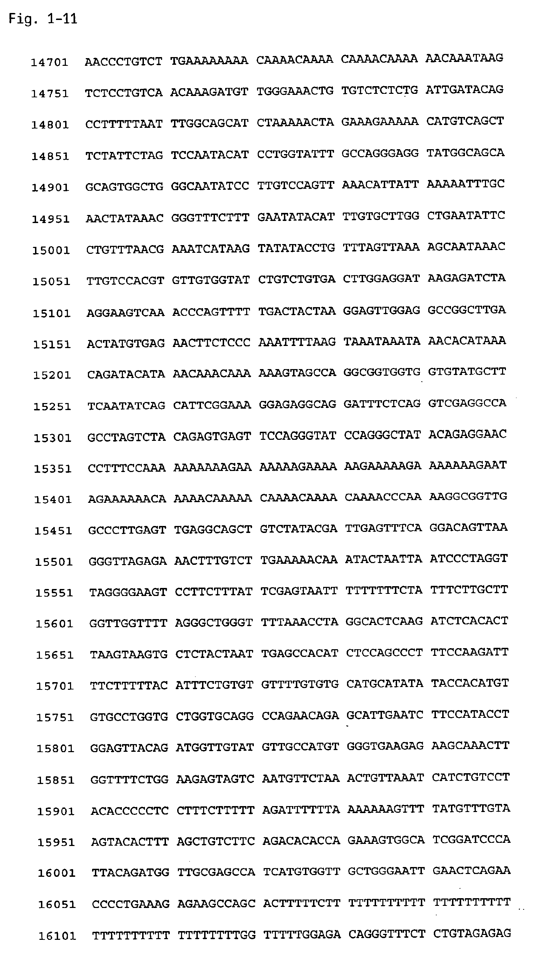

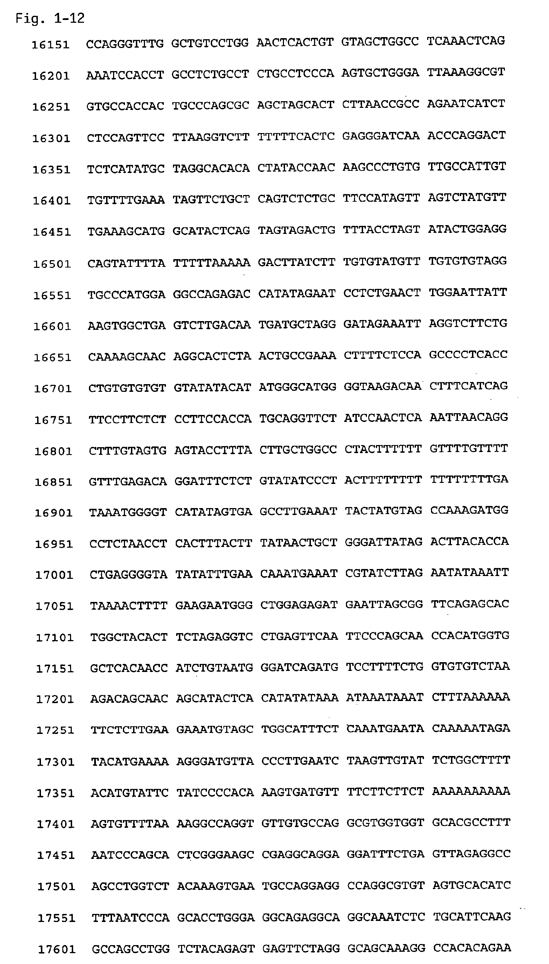

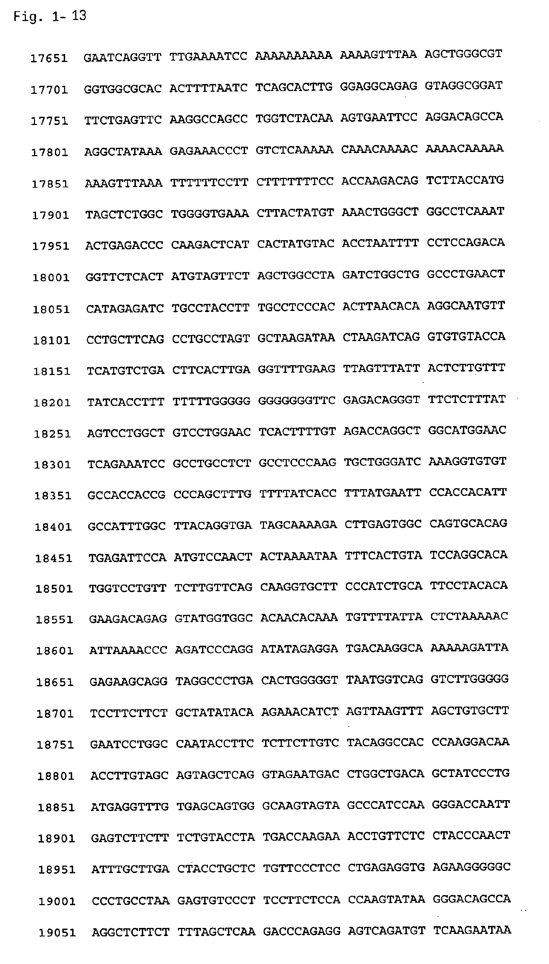

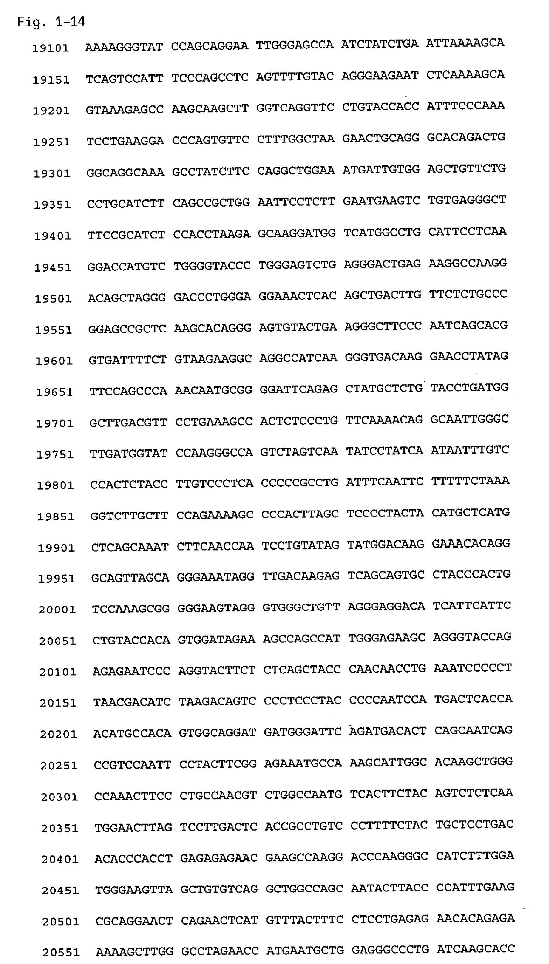

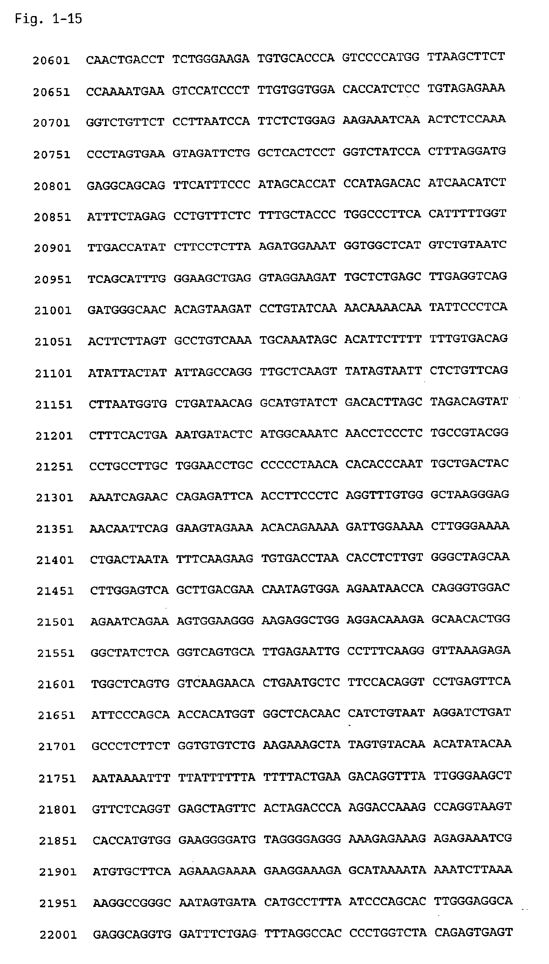

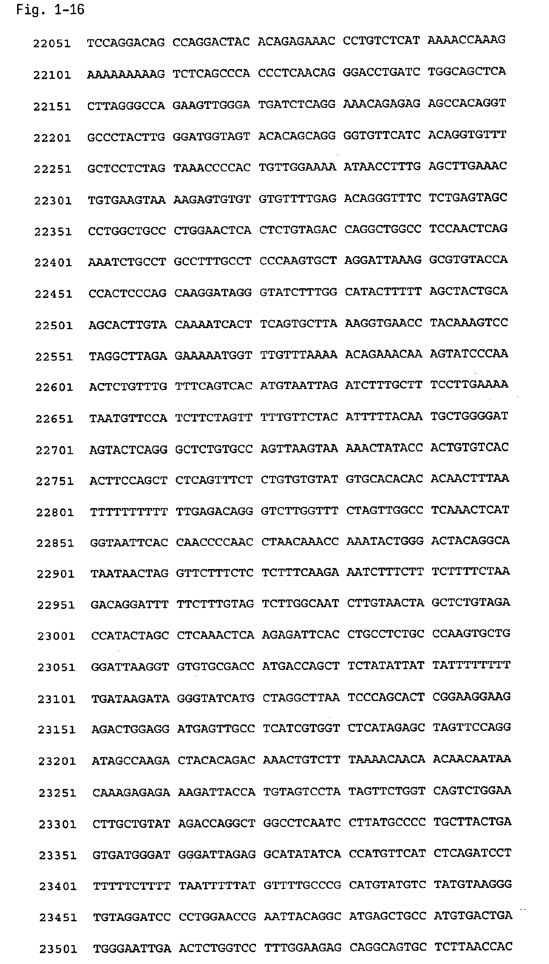

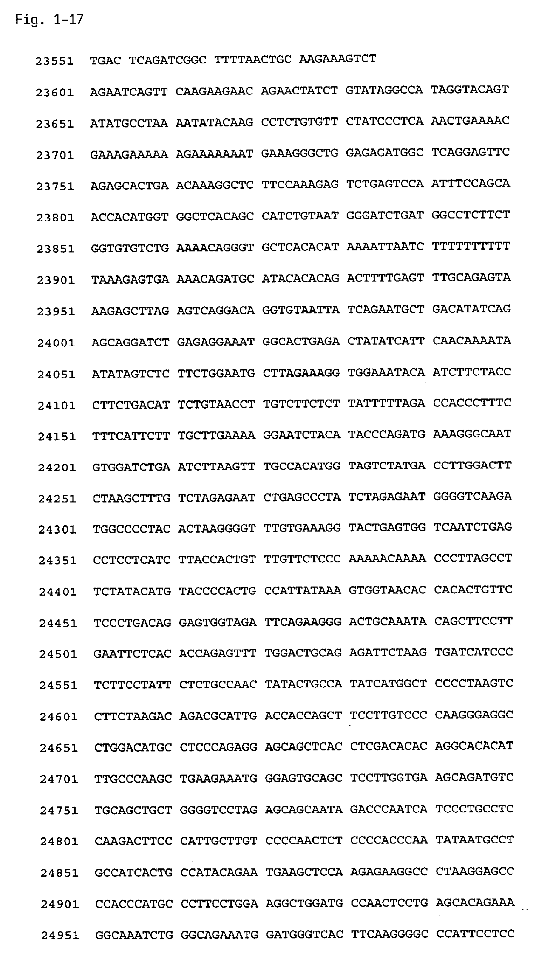

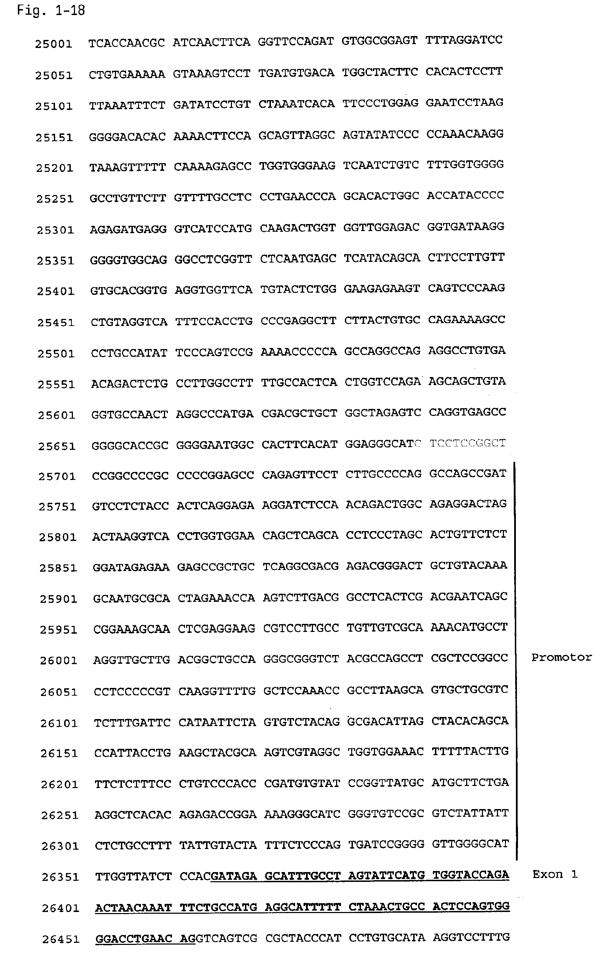

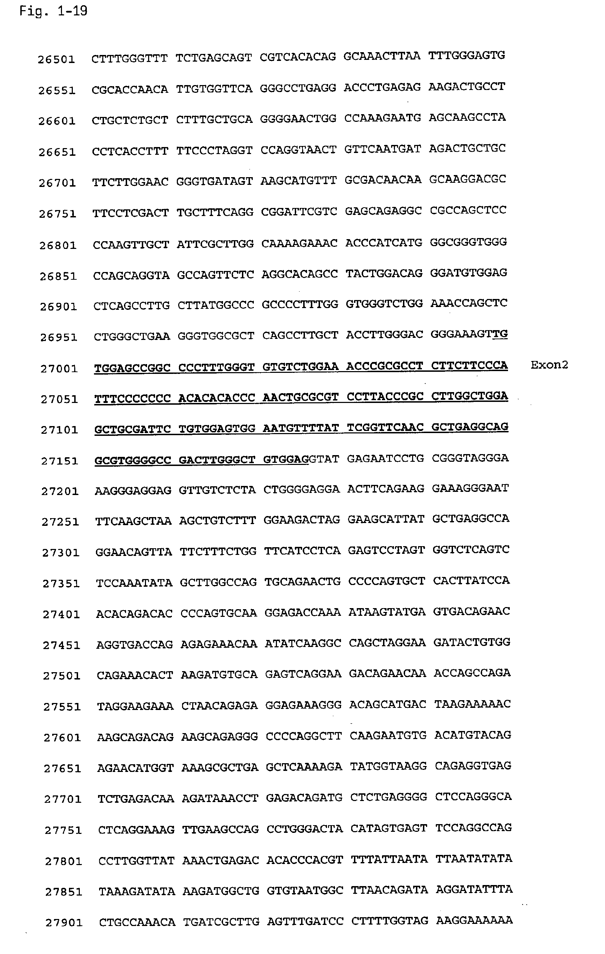

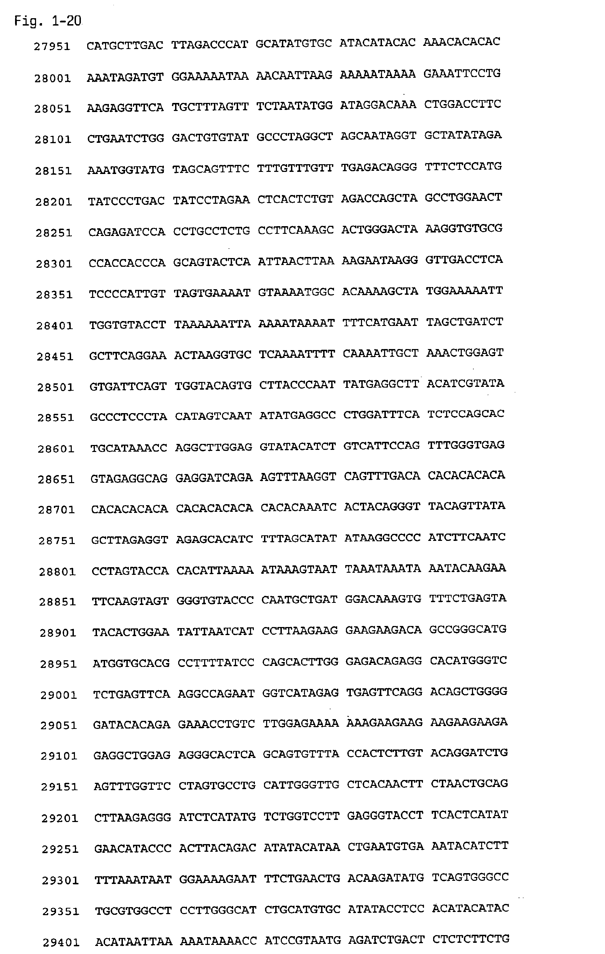

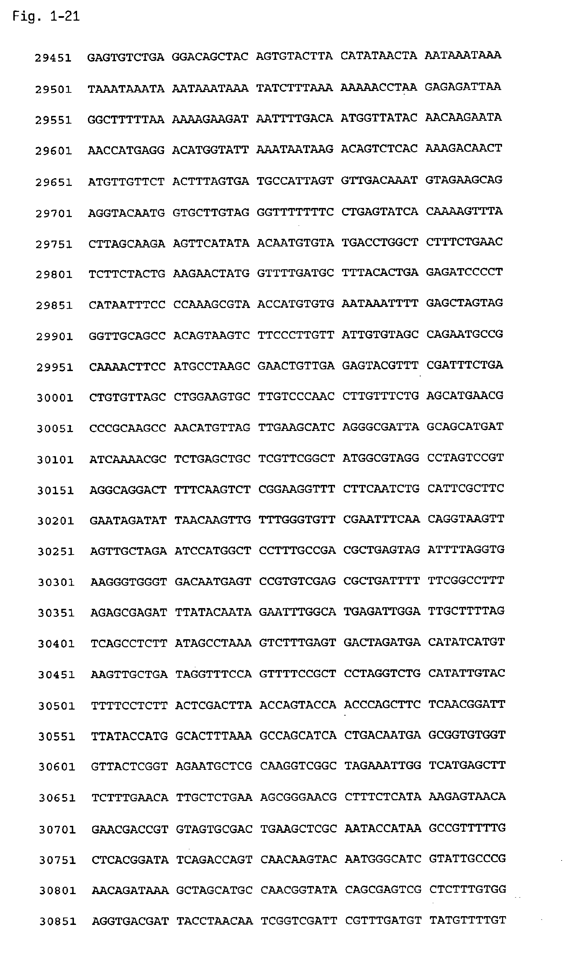

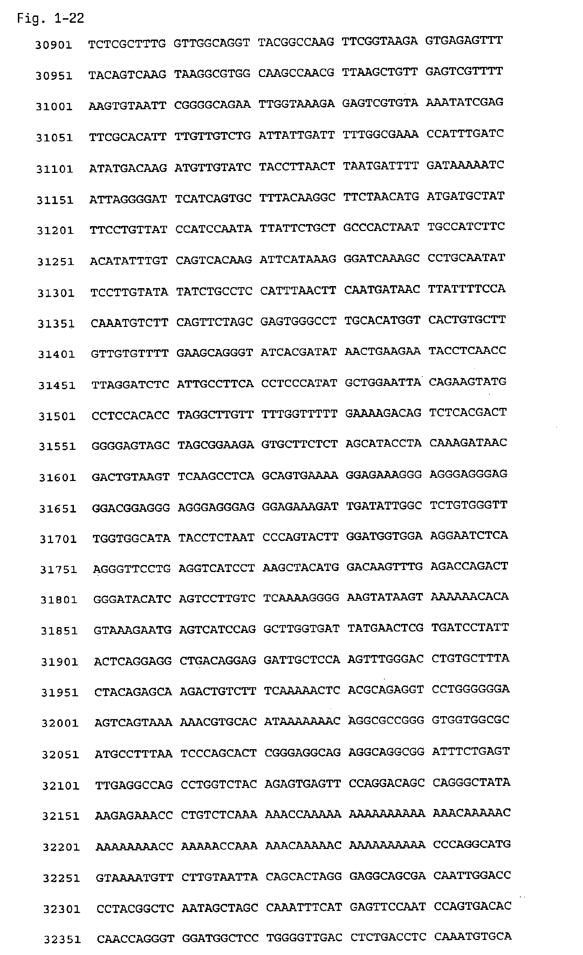

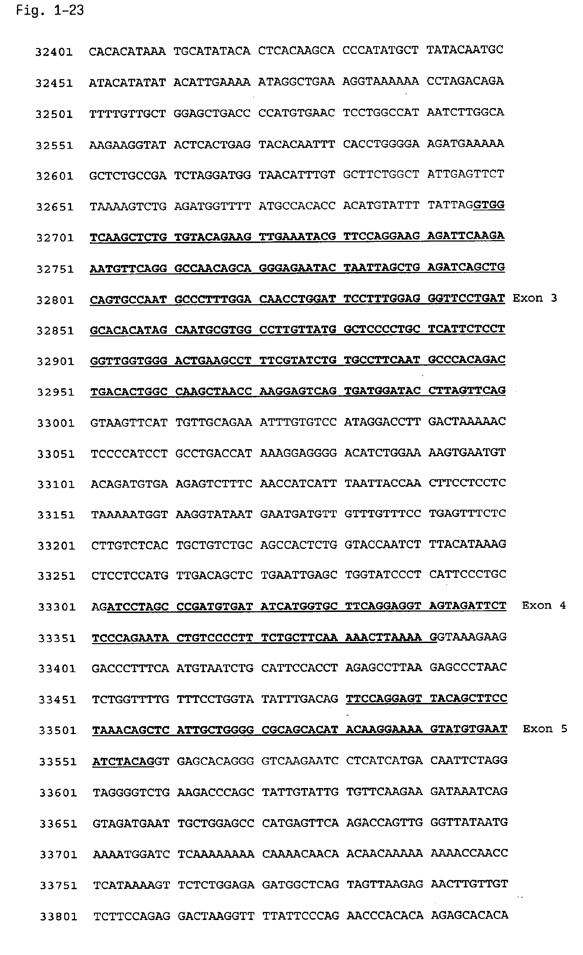

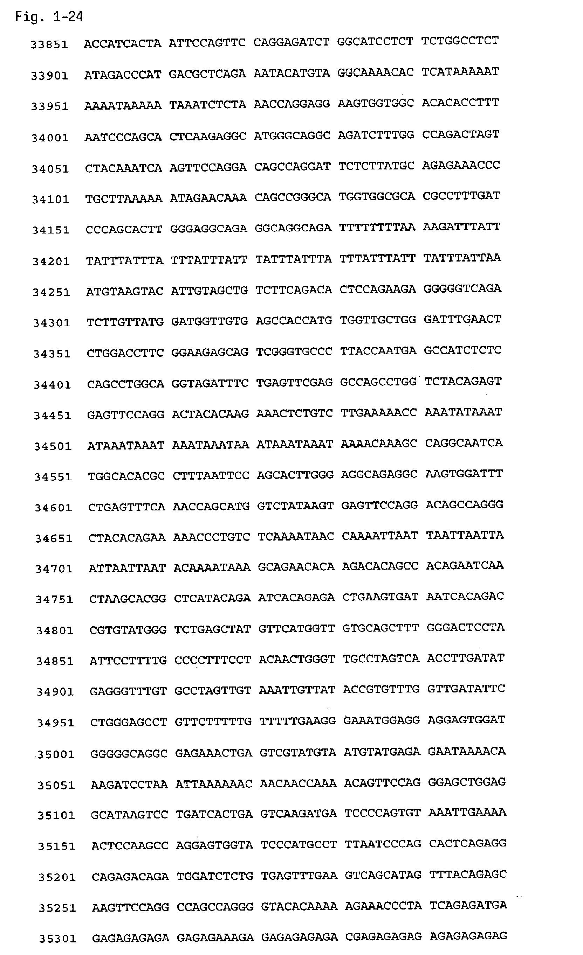

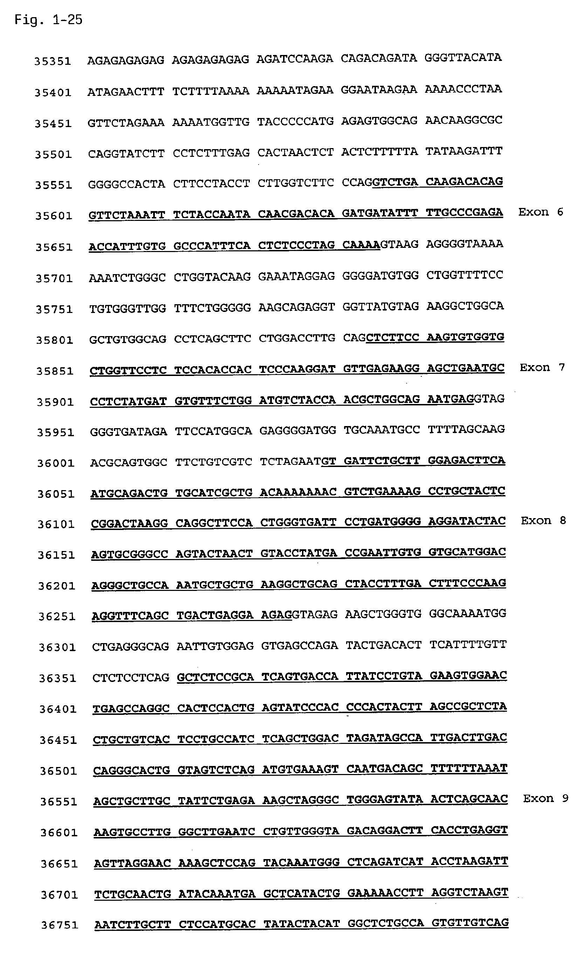

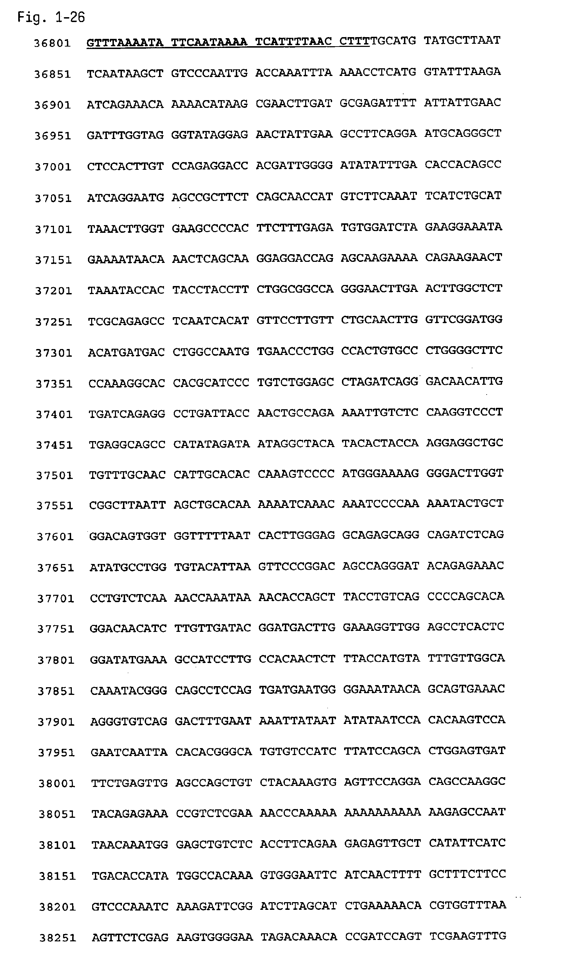

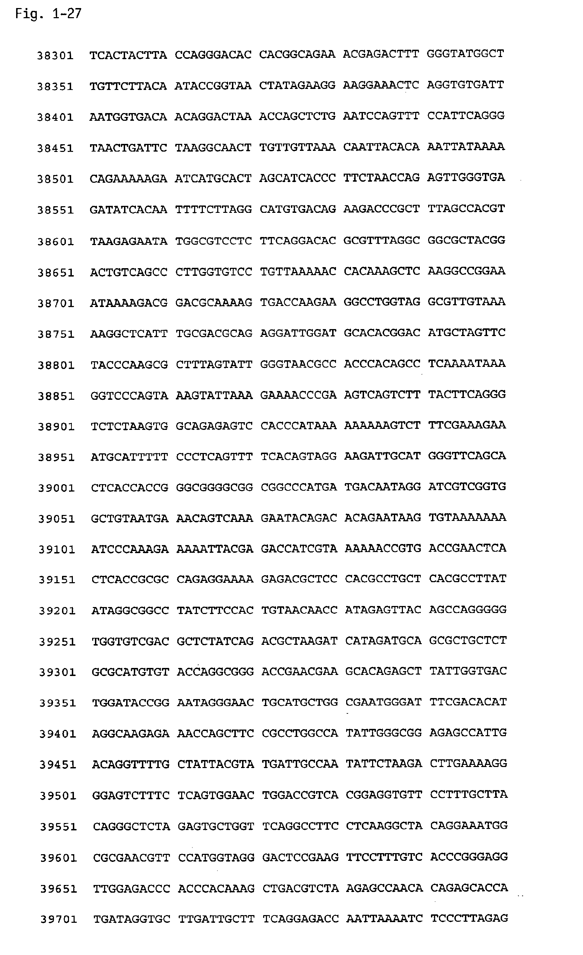

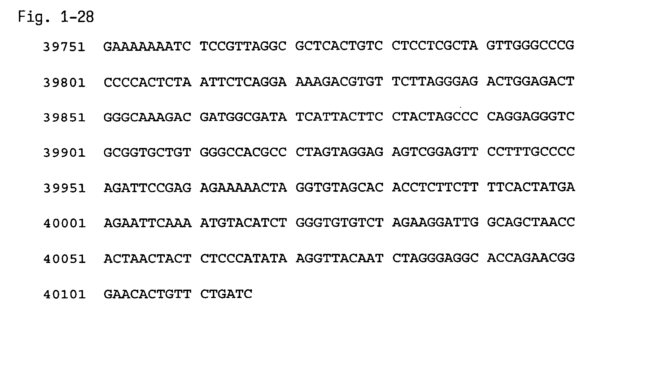

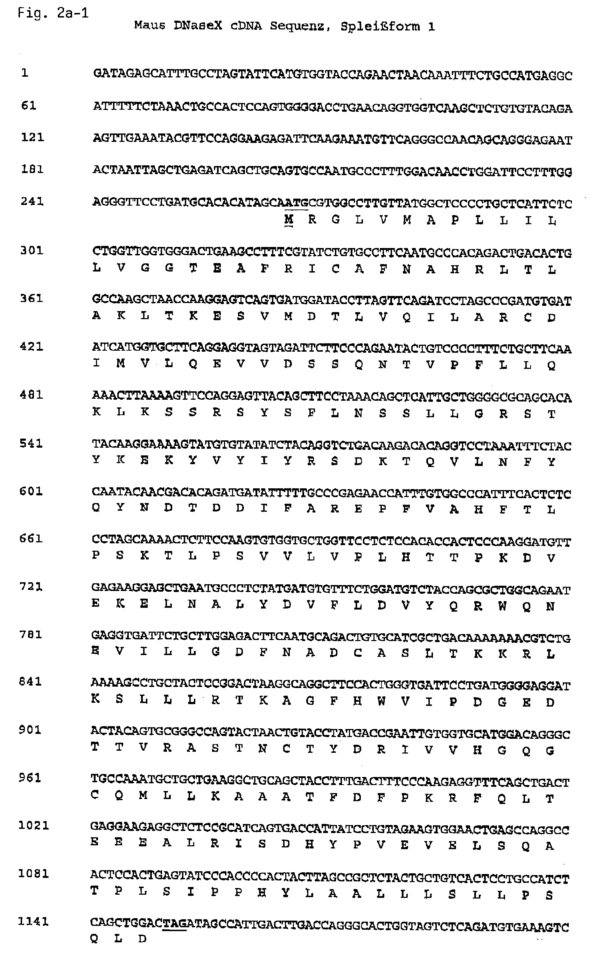

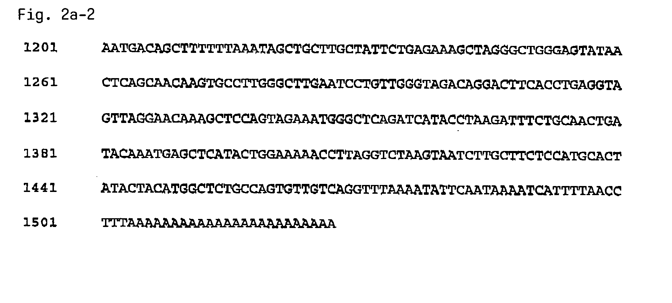

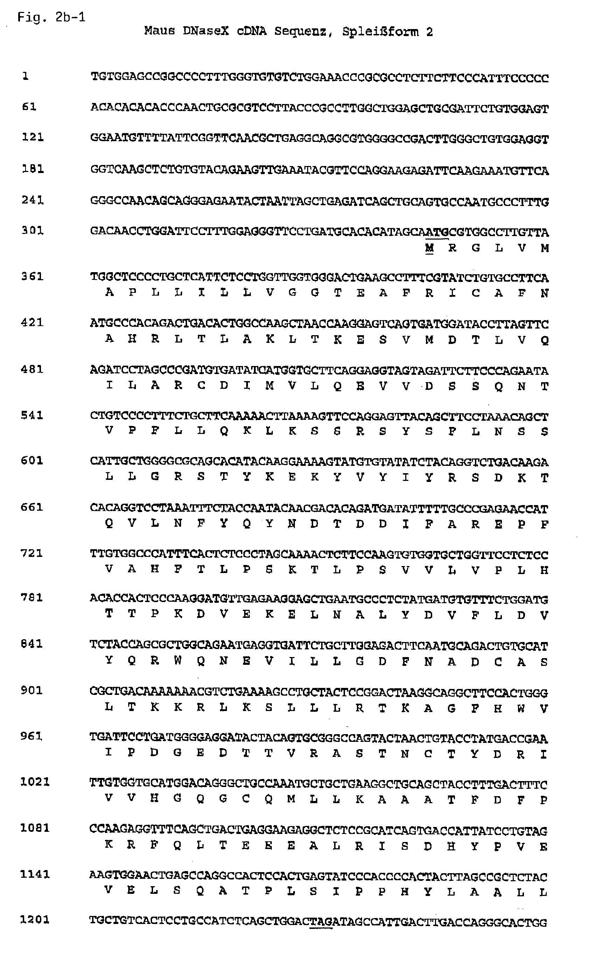

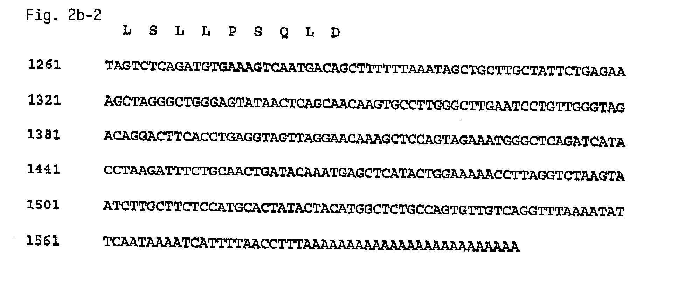

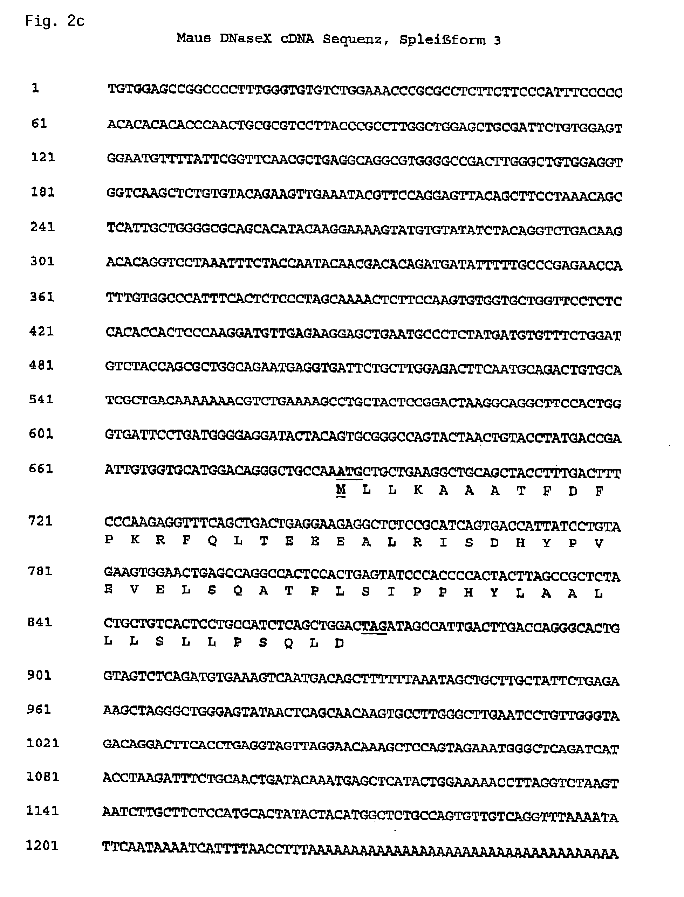

Gegenstand der vorliegenden Erfindung ist somit eine DNA-Sequenz, die die in Figur 1 gezeigte DNA-Sequenz (genomische DNA) umfaßt. Von muriner DNase X gibt es drei Spleißvarianten (Fig. 2a, 2b, 2c), von denen mit großer Wahrscheinlichkeit Spleißform 1 und 2 aktiv sind, während Spleißform 3 als inaktiv anzusehen ist. Die vorliegende Erfindung betrifft ferner eine DNA-Sequenz, die ein Protein mit den biologischen Eigenschaften einer DNase X kodiert, wobei diese DNA-Sequenz ein Fragment, eine allelische Variante oder eine andere Variante der vorstehenden erfindungsgemäßen DNA-Sequenzen ist. Außerdem betrifft die vorliegende Erfindung nicht transkribierte und transkribierte Steuerungselemente. Eine nicht transkribierte Steuerungssequenz stellt der DNase X-Promotor dar. Dieser kann u.a. dazu einsetzt werden, eine DNase-spezifische Expression eines beliebigen Proteins zu gewährleisten. Beispielsweise kann dadurch eine genetisch veränderte Mauslinie generiert werden, bei der das Cre-Protein (Cre-Rekombinase) unter der Kontrolle des DNase X-Promotors liegt. Die vorliegende Erfindung betrifft weiterhin eine transkribierte, nicht-translatierte differentiell gespleißte DNase X-Sequenz im 5'-Bereich des DNase X-Transkripts. Diese Sequenz kann für die Steuerung und Aktivierung des DNase X-Proteins verwendet werden. Desweiteren kann ein beliebiges Protein unter die Kontrolle dieses Steuerungselements gestellt werden. Weiterhin können an das DNase X-Transkript bindende Proteine isoliert werden. Hierdurch können unter anderem die Translationseffizienz des DNase X-Transkriptes gezielt durch an das Transkript bindende Proteine beeinflußt werden.

Die in der vorliegenden Erfindung verwendeten Begriffe "Variante" oder "Fragment" umfassen DNA-Sequenzen, die sich gegenüber den in Figur 1 angegebenen Sequenzen durch Deletion(en), Insertion(en) oder Austausch(e) und/oder andere im Stand der Technik bekannte Modifikationen unterscheiden bzw. ein Fragment des ursprünglichen DNA-Moleküls umfassen, wobei das durch diese DNA-Sequenzen kodierte Protein noch die biologischen Eigenschaften des DNase X-Proteins aufweist und in Säugern biologisch aktiv ist. Dazu zählen auch Allelvarianten. Die vorstehenden Veränderungen in der DNA-Sequenz führen zu einem Protein, das zu 90%, vorzugsweise 95%, mehr bevorzugt 98% und noch mehr bevorzugt 99% mit dem der murinen DNase X identisch ist. Am meisten bevorzugt sind Veränderungen in der DNA-Sequenz, die lediglich zu konservativen Aminosäureaustauschen führen. Verfahren zur Erzeugung der vorstehenden Änderungen in der DNA-Sequenz sind dem Fachmann bekannt und in Standardwerken der Molekularbiologie beschrieben, beispielsweise in Sambrook et al., Molecular Cloning: A Laboratory Manual, 2. Ausgabe, Cold Spring Harbor Laboratory Press, Cold Spring Harbor NY (1989). Der Fachmann ist auch in der Lage zu bestimmen, ob eine von einer so veränderten DNA-Sequenz kodierte DNase X noch über die biologischen Eigenschaften von DNase X verfügt. Nachstehend werden alle diese DNA-Sequenzen allgemein mit dem Begriff "DNase X-Sequenzen" bezeichnet.

DNase X-Sequenzen können auch in einen Vektor, vorzugsweise Expressionsvektor, inseriert werden. Somit umfaßt die vorliegende Erfindung auch diese Vektoren bzw. Expressionsvektoren. Die Bezeichnung "Vektor" bezieht sich auf ein Plasmid (pUC18, pBR322, pBlueScript etc.), auf ein Virus oder ein anderes geeignetes Vehikel. In einer bevorzugten Ausführungsform ist die erfindungsgemäße DNase X-Sequenz im Vektor mit regulatorischen Elementen funktionell verknüpft, die dessen Expression in prokaryotischen oder eukaryotischen Wirtszellen erlauben. Solche Vektoren enthalten neben den regulatorischen Elementen, beispielsweise einem Promotor, typischerweise einen Replikationsursprung und spezifische Gene, die die phänotypische Selektion einer transformierten Wirtszelle erlauben. Zu den regulatorischen Elementen für die Expression in Prokaryoten, beispielsweise E.coli, zählen der lac-,trp-Promotor oder T7-Promotor, und für die Expression in Eukaryoten der AOX1- oder GAL1-Promotor in Hefe und der CMV-,SV40-,RVS-40-Promotor, CMV- oder SV40-Enhancer für die Expression in tierischen Zellen. Geeignete regulatorische Sequenzen sind außerdem beschrieben in Goeddel: Gene Expression Technology: Methods in Enzymology 185, Academic Press, San Diego, CA (1990). Weitere Beispiele für geeignete Promotoren sind der Metallothionein I- und der Polyhedrin-Promotor. Zu geeigneten Expressionsvektoren für E.coli zählen beispielsweise pGEMEX, pUC-Derivate, pGEX-2T, pET3b und pQE-8. Zu den für die Expression in Hefe geeigneten Vektoren zählen py100 und Ycpad1, für die Expression in Säugerzellen pMSXND, pKCR, pEFBOS, cDM8 und pCEV4 sowie von pcDNAl/amp pcDNAl/neo, pRc/CMV, pSV2gpt, pSV2neo, pSV2-dhfr, pTk2, pRSVneo, pMSG, pSVT7, pko-neo und pHyg stammende Vektoren. Zu den erfindungsgemäßen Expressionsvektoren zählen auch von Baculovirus abgeleitete Vektoren für die Expression in Insektenzellen, beispielsweise pAcSGHisNT-A.

Vorzugsweise werden die vorstehend beschriebenen DNase X-Sequenzen in einen für die Gentherapie geeigneten Vektor inseriert, beispielsweise unter Kontrolle eines gewebespezifischen Promotors, und in die Zellen eingeschleust. In einer bevorzugten Ausführungsform ist der die vorstehend beschriebenen DNase X-Sequenzen enthaltende Vektor ein Virus, beispielsweise ein Adenovirus, Vaccinia-Virus oder Retrovirus. Besonders bevorzugt sind Retroviren. Beispiele für geeignete Retroviren sind MoMuLV, HaMuSV, MuMTV, RSV oder GaLV. Für die Gentherapie geeignete Vektoren sind außerdem in WO 93/04701, WO 92/22635, WO 92/20316, WO 92/19749 und WO 92/06180 offenbart. Für Zwecke der Gentherapie können die vorstehend beschriebenen DNase X-Sequenzen auch in Form von kolloidalen Dispersionen zu den Zielzellen transportiert werden. Dazu zählen beispielsweise Liposomen oder Lipoplexe (Mannino et al., Biotechniques 6 (1988), 682).

Allgemeine, auf dem Fachgebiet bekannte Verfahren können zur Konstruktion von Expressionsvektoren, die DNase X-Sequenzen und geeignete Kontrollsequenzen enthalten, verwendet werden. Zu diesen Verfahren zählen beispielsweise in vitro-Rekombinationstechniken, synthetische Verfahren, sowie in vivo-Rekombinationsverfahren, wie sie beispielsweise in Sambrook et al., supra, beschrieben sind. Die erfindungsgemäßen DNase X-Sequenzen können auch in Verbindung mit einer für ein anderes Protein bzw. Peptid kodierenden DNA inseriert werden, so daß DNase X beispielsweise in Form eines Fusionsproteins exprimiert werden kann. Der Fachmann kennt auch Verfahren zur rekombinanten Herstellung von DNase X, d.h. die Kultivierung geeigneter Wirtszellen unter Bedingungen, die die Expression des Proteins (bzw. Fusionsproteins) erlauben (vorzugsweise stabile Expression), und die Gewinnung des Proteins aus der Kultur oder aus den Wirtszellen. Geeignete Reinigungsverfahren (beispielsweise präparative Chromatographie, Affinitätschromatographie, beispielsweise Immunoaffinitätschromatographie, HPLC etc.) sind ebenfalls allgemein bekannt.

Die vorliegende Erfindung betrifft auch ein murines DNase X-Protein oder ein Protein mit dessen biologischer Aktivität, das von den erfindungsgemäßen DNase X-Sequenzen kodiert wird. Es existieren drei Spleißformen von DNase X-Protein, die in den Figuren 2a, 2b und 2c gezeigt sind.

Die erfindungsgemäßen DNA-Sequenzen und Protein-Sequenzen eignen sich auch dafür geeignete Bindungspartner zu finden, mit denen über die Funktion des DNase-Gens weitere Aussagen getroffen werden können. Desweiteren eignen sich diese Bindungspartner dazu, ggf. als Arzneimittel verabreicht zu werden.

Außerdem betrifft die vorliegende Erfindung einen Antikörper, der gegen ein von den erfindungsgemäßen DNase X-Sequenzen kodiertes DNase X-Protein gerichtet ist. Diese Antikörper können monoklonale, polyklonale oder synthetische Antikörper sein oder Fragmente davon. In diesem Zusammenhang bedeutet der Begriff "Fragment" alle Teile des monoklonalen Antikörpers (z.B. Fab-, Fv- oder "single chain Fv"-Fragmente (scFv)), welche die gleiche Epitopspezifität wie der vollständige Antikörper aufweisen. Die Herstellung solcher Fragmente ist dem Fachmann bekannt. Vorzugsweise handelt es sich bei den erfindungsgemäßen Antikörpern um monoklonale Antikörper. Die erfindungsgemäßen Antikörper können gemäß Standardverfahren hergestellt werden, wobei vorzugsweise die von den erfindungsgemäßen DNase X-Sequenzen kodierte DNase X oder ein (synthetisches) Fragment davon als Immunogen dienen. Verfahren zur Gewinnung monoklonaler Antikörper sind dem Fachmann bekannt. Dazu zählt auch die Isolierung des gewünschten Antikörpers, z.B. eines scFv-Fragments, aus komplexen Antikörper-Banken über Phagen-Display.

Es kann davon ausgegangen werden, daß bei Erkrankungen, bei denen Apoptose eine Rolle spielt, insbesondere SLE, die Konzentration oder Aktivität von DNase X im Vergleich zur Normalsituation verändert, d.h. in der Regel verringert, ist. Somit betrifft die vorliegende Erfindung auch ein Diagnoseverfahren zum Nachweis einer veränderten Expression oder Konzentration von DNase X, bei dem man eine Probe mit einer zur spezifischen Hybridisierung mit einer von einer DNase X-Sequenz transkribierten mRNA geeigneten Sonde oder einem Primer bzw. einem Primersatz oder einem anti-DNase X-Antikörper in Berührung bringt und sodann direkt oder indirekt bestimmt, ob sich die Konzentration von DNase X-mRNA oder DNase X-Protein in der Probe im Vergleich zu einer Kontrollprobe (von einer gesunden Person) unterscheiden. Der Fachmann kennt Verfahren zur geeigneten Probeentnahme und auch geeignete Kontrollproben. Vorzugsweise handelt es sich bei der Kontrollprobe um ein Gewebe, das dem gleichen Gewebe wie bei dem SLE-Patienten entspricht, jedoch von einer gesunden Quelle stammt. Eine im Vergleich zu der Kontrollprobe verringerte DNase X-Aktivität bzw. Konzentration ist ein diagnostisches Anzeichen für SLE oder eine Prädisposition für SLE. Entsprechendes gilt für die übrigen Erkrankungen. Die für dieses Diagnoseverfahren verwendbaren Sonden umfassen auf den erfindungsgemäßen DNase X-Sequenzen basierende Primer, beispielsweise für eine PCR, RT-PCR oder eine Aptamerdiagnostik (SELEX-Verfahren). Vorzugsweise sind die Sonden (oder Primer) Oligonukleotide, die einen Nukleinsäure-Bereich umfassen, der unter stringenten Hybridisierungsbedingungen (gemäß der Definition in Sambrook, supra) mit mindestens 13, vorzugsweise mindestens 20 aufeinanderfolgende Nukleotiden der Sequenz von Figur 2a oder 2b oder natürlich vorkommenden Varianten davon hybridisiert. Die Sonde (bzw. der Primer) kann auch eine Markierung tragen, z.B. ein Radioisotop, fluoreszierende Verbindung, Enzym oder Enzym-Cofaktor. Der Fachmann kennt auch Bedingungen, die es erlauben, daß bei der PCR etc. nur die DNase X-mRNA nicht jedoch die DNase X-DNA amplifiziert wird bzw. zwischen DNA-Amplifikationsprodukten und mRNA-Amplifikationsprodukten unterschieden werden kann, z.B. durch Behandlung der Probe mit DNAse oder durch Verwendung von Primern, die zur Amplifikation von Intron-Bereichen führen, somit eine DNA-Amplifikat im Vergleich zum mRNA-Amplifikat länger ist. Alternativ kann auch ein oligo(dT)-verankerter Primer für PCR, RT-PCR etc. verwendet werden.

Schließlich betrifft die vorliegende Erfindung einen diagnostischen Kit zur Durchführung des erfindungsgemäßen Diagnoseverfahrens, der eine zur spezifischen Hybridisierung mit einer von einer DNase X-DNA-Sequenz transkribierten mRNA geeignete Sonde oder einen Primer/Primersatz und/oder einen anti-DNase X-Antikörper oder ein Fragment davon enthält. Je nach Ausgestaltung des diagnostischen Kits können die Probe oder die Sonde bzw. der Primer bzw. der Antikörper oder das Fragment davon immobilisiert sein. Die Antikörper können beispielsweise in Immunassays auch in Flüssigphase vorliegen. Dabei können die Antikörper auf verschiedene Art und Weise markiert sein. Geeignete Marker und Markierungsverfahren sind auf dem Fachgebiet bekannt. Beispiele für Immunassays sind ELISA und RIA.

Die vorliegende Erfindung betrifft auch ein Arzneimittel, das eine erfindungsgemäße DNase X-Sequenz, einen erfindungsgemäßen Expressionsvektor oder ein erfindungsgemäßes DNase X-Protein enthält. Das erfindungsgemäße Arzneimittel erlaubt die Durchführung von präventiven oder therapeutischen Maßnahmen hinsichtlich Erkrankungen, bei denen Apoptose eine Rolle spielt, insbesondere hinsichtlich SLE. Vorzugsweise ist das Arzneimittel mit einem geeigneten Träger kombiniert. Geeignete Träger und die Formulierung derartiger Arzneimittel sind dem Fachmann bekannt. Zu geeigneten Trägern zählen beispielsweise Phosphat-gepufferte Kochsalzlösungen, Wasser, Emulsionen, beispielsweise Öl/Wasser-Emulsionen, Netzmittel, sterile Lösungen etc. Die Verabreichung der Arzneimittel kann oral oder parenteral erfolgen. Zu den Verfahren für die parenterale Verabreichung gehören die topische, intra-arterielle, intramuskuläre, subkutane, intramedulläre, intrathekale, intraventrikuläre, intravenöse, intraperitoneale oder intranasale Verabreichung.

Die vorliegende Erfindung betrifft schließlich auch ein nicht-menschliches Säugetier, bei dem das DNase X-Gen, das die erfindungsgemäßen DNase X-Sequenzen enthält, ubiquitär oder gewebespezifisch verändert, vorzugsweise inaktiviert ist ("knock-out"). Ein solches nicht-menschliches Säugetier, vorzugsweise eine Maus, kann durch übliche Verfahren bereitgestellt werden. Günstig ist ein Verfahren, das folgende Schritte umfaßt:
(a) Herstellung eines DNA-Fragments, insbesondere eines Vektors, der ein verändertes murines DNase X-Gen enthält, wobei das DNase X-Gen durch Insertion einer heterologen Sequenz, insbesondere eines selektierbaren Markers, verändert worden ist;
(b) Präparation embryonaler Stammzellen aus einem nicht-menschlichen Säuger (bevorzugt Maus);
(c) Transformation der embryonalen Stammzellen von Schritt (b) mit dem DNA-Fragment von Schritt (a), wobei das DNase X-Gen in den embryonalen Stammzellen durch homologe Rekombination mit dem DNA-Fragment von (a) verändert wird;
(d) Kultivieren der Zellen von Schritt (c),
(e) Selektion der kultivierten Zellen von Schritt (d) auf das Vorhandensein der heterologen Sequenz, insbesondere des selektierbaren Markers,
(f) Erzeugen chimärer nicht-menschlicher Säuger aus den Zellen von Schritt (e) durch Injektion dieser Zellen in Säuger-Blastozysten (bevorzugt Maus-Blastozysten); und
(g) Übertragen der in Schritt (f) erhaltenen Blastozysten in pseudo-schwangere weibliche Säuger (bevorzugt Maus) und Analyse der erhaltenen Nachkommen hinsichtlich eines veränderten, insbesondere inaktivierten DNase X-Gens.

In Schritt (c) wird der Mechanismus der homologen Rekombination (vgl. R.M. Torres, R. Kühn, Laboratory Protocols for Conditional Gene Targeting, Oxford University Press, 1997) ausgenutzt, um embryonale Stammzellen zu transfizieren. Die homologe Rekombination zwischen den in einem Chromosom vorhandenen DNase X-Sequenzen und neuen, hinzugefügten klonierten DNA-Sequenzen ermöglicht das Einfügen eines klonierten Gens in das Genom einer lebenden Zelle anstelle des ursprünglichen Gens. Mit dieser Methode können bei Verwendung embryonaler Keimzellen via Chimären Tiere erhalten werden, die für das gewünschte Gen oder den gewünschten Genteil oder die gewünschte Mutation homozygot sind. Die Herstellung transgener Säugetiere ist außerdem in A. L. Joyner Ed., Gene Targeting, A Practical Approach (1993), Oxford University Press, beschrieben.

Der Ausdruck "embryonale Stammzellen" betrifft jegliche embryonalen Stammzellen eines nicht-menschlichen Säugetiers, die sich zur Mutierung des DNase X-Gens eignen. Vorzugsweise sind die embryonalen Stammzellen von der Maus, insbesondere die Zellen E14/1 oder 129/SV.

In diesem Zusammenhang umfaßt der Ausdruck "Vektor" jeglichen Vektor, der durch Rekombination mit der DNA von embryonalen Stammzellen eine Veränderung des DNase X-Gens ermöglicht. Vorzugsweise weist der Vektor einen Marker auf, mit dem auf vorhandene Stammzellen selektioniert werden kann, in denen die gewünschte Rekombination erfolgt ist. Ein solcher Marker ist z.B. die IoxP/tk neo-Cassette, die mit Hilfe des Cre/loxP-Systems wieder aus dem Genom entfernt werden kann (Sauer et al., Proc. Natl. Acad. Sci. USA 85 (14), S. 5166-5179, 1988). Desweiteren kennt der Fachmann Bedingungen und Materialien, um die Schritte (a)-(g) durchzuführen.

Mit der vorliegenden Erfindung wird ein nicht-menschliches Säugetier, vorzugsweise eine Maus, bereitgestellt, dessen DNase X-Gen verändert ist. Diese Veränderung kann z.B. ein Ausschalten der Funktion sein. Mit einem solchen Säugetier bzw. Zellen daraus kann selektiv die Funktion des DNase X-Proteins untersucht werden. Ferner ist es hiermit möglich, Substanzen, Arzneimittel und Therapieansätze zu finden, mit denen selektiv auf die Funktion der DNase X eingewirkt werden kann. Daher liefert die vorliegende Erfindung eine Basis, um auf Erkrankungen, bei denen Apoptose eine Rolle spielt (z.B SLE) einzuwirken. Ferner ermöglicht das erfindungsgemäße Säugetier mit veränderter DNase X-Funktion Untersuchungen, ob man durch Applikation von diversen Verbindungen einen abgeschwächten Verlauf der Erkrankungen bzw. eine Heilung in diesen Tieren beobachtet. Dieses Tier stellt somit ein neues Krankheitsmodell für die Entwicklung neuer bzw. weiterer Therapieansätze dar. Mit dem erfindungsgemäßen nicht-menschlichen Säugetier ist es möglich, die Auswirkungen einer, z.B. auch gewebespezifischen, Veränderung bzw. Inaktivierung des DNase X-Gens zu untersuchen, beispielsweise hinsichtlich der möglichen Veränderung der Expression weiterer Gene, die mit der Entwicklung von z.B. SLE in Zusammenhang stehen können. Die Kenntnis dieser Gene und deren Funktion erlaubt dann auch die Entwicklung weiterer Ansatzpunkte für die pharmakologische Beeinflussung von z.B. SLE oder zystischer Fibrose oder zu den Erkrankungen führenden Prozessen, beispielsweise durch die Bereitstellung von Verbindungen, die die Expression dieser Gene oder die Aktivität der Genprodukte spezifisch beeinflussen.

Somit betrifft die vorliegende Erfindung auch die Verwendung des erfindungsgemäßen nicht-menschlichen Säugetiers zur weiteren Erforschung der Erkrankungen oder zur Charakterisierung von Genen und/oder Testung von Substanzen, Arzneimitteln und/oder Therapieansätzen, die mit den Erkrankungen, bei denen Apoptose eine Rolle spielt, oder zu den Erkrankungen führenden Prozessen, insbesondere SLE, in Zusammenhang stehen.

Die vorliegende Erfindung ist von großem Nutzen für die Entwicklung eines Agens für die Diagnose, Prävention oder Therapie von Krebs, Autoimmunkrankheiten, AIDS und Erkrankungen, bei denen Apoptose eine Rolle spielt. Durch die Aktivierung der vorliegenden DNase X ist es möglich, unerwünschte Zellen durch die gezielte Auslösung von Apoptose zu entfernen. Hierzu zählen Krebszellen und neuronale Zellen, die neurodegenerative Prozesse verursachen. Durch die Inhibierung der vorliegenden DNase oder der durch DNase gesteuerten Prozesse können gezielt Zellen vor dem programmierten Zelltod geschützt werden. Diese können dann den gewünschten Effekt auf den Organismus ausüben. Durch die Applikation von muriner DNase X kann desweiteren Schleim in der Lunge von Patienten mit zystischer Fibrose gelöst und abgebaut werden. Bei Patienten mit systemischem Lupus Erythematosus kann DNase X appliziert werden und die immunogenen DNA-Komplexe können abgebaut werden und somit eine Immunreaktion verhindert werden.

### Kurze Beschreibung der Figuren:

### Figur 1: Genomische Sequenz der murinen DNase X

### Figur 2: (a) Spleißform 1 von muriner DNase X, (b) Spleißform 2 von muriner DNase X, (c) Spleißform 3 von muriner DNase X.

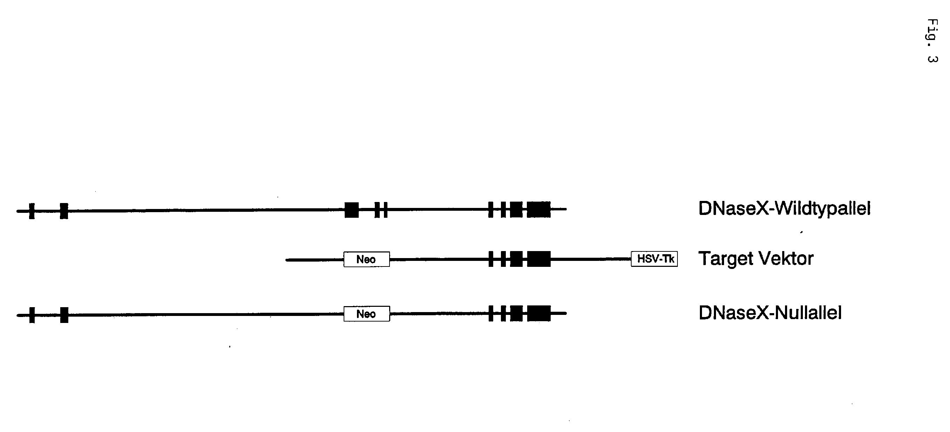

### Figur 3: Genstruktur des DNase X-Gens

Die gezeigte Verteilung der Exons und Introns repräsentiert das DNase X Wildtypallel. Bei der Herstellung des Target-Vektors wurden drei kodierende Exons entfernt und durch die neo-Kassette ersetzt. Durch eine homologe Rekombination in ES-Zellen wurde ein DNase X-Nullallel generiert, bei dem wesentliche Teile des kodierenden Bereichs deletiert sind.

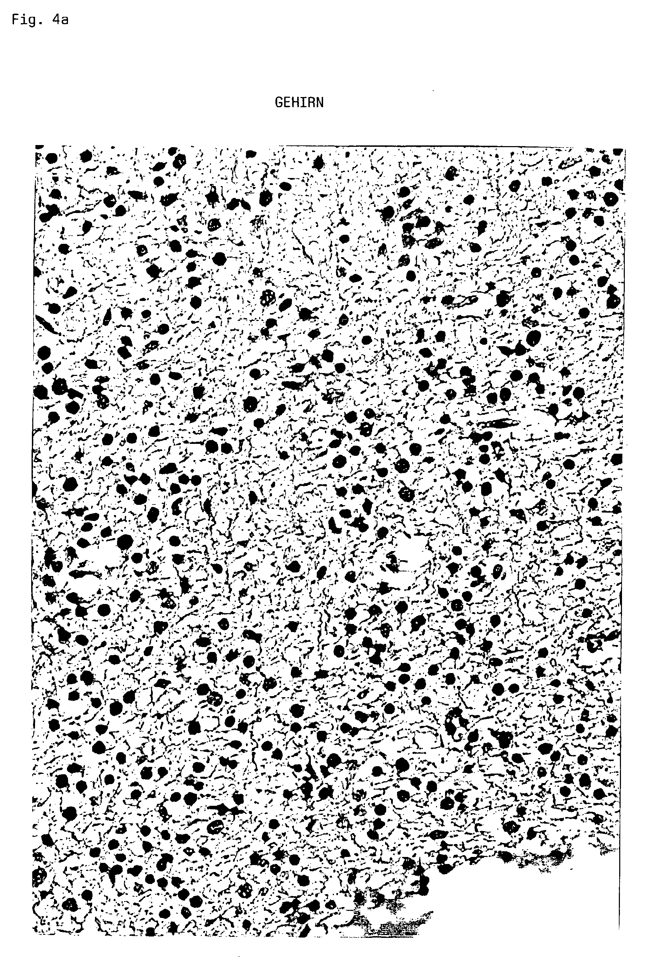

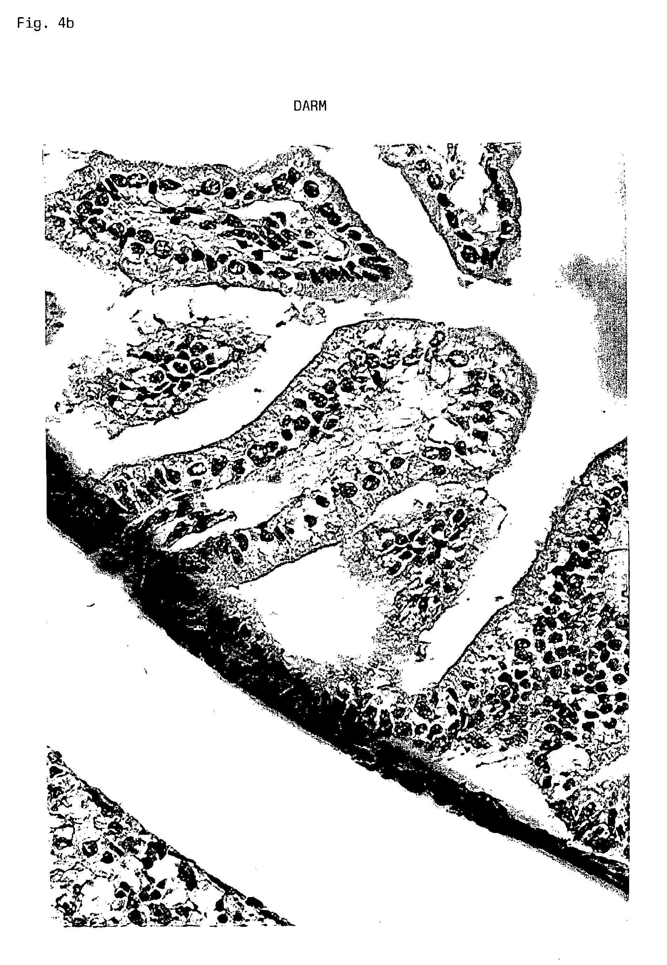

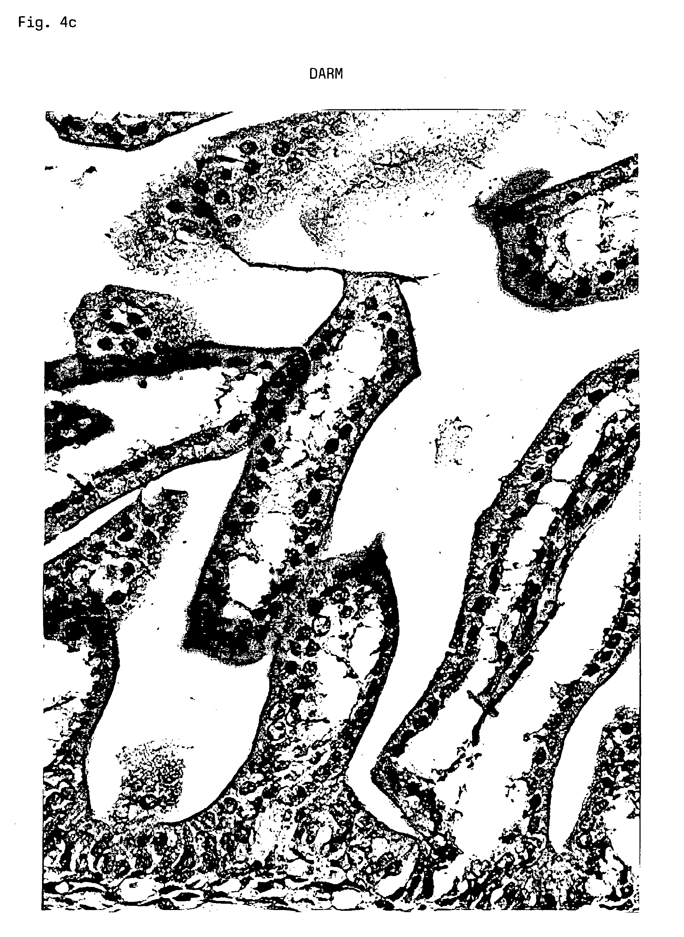

### Figur 4: Immunhistochemische Analyse des murinen DNase X-Proteins

A: Expression des DNase X-Proteins in einer Subpopulation von zellen im Gehirn des Mausembryos (E17,5)
B + C: Expression des DNase X-Proteins im Darmepithel eines Mausembryos (E17,5)

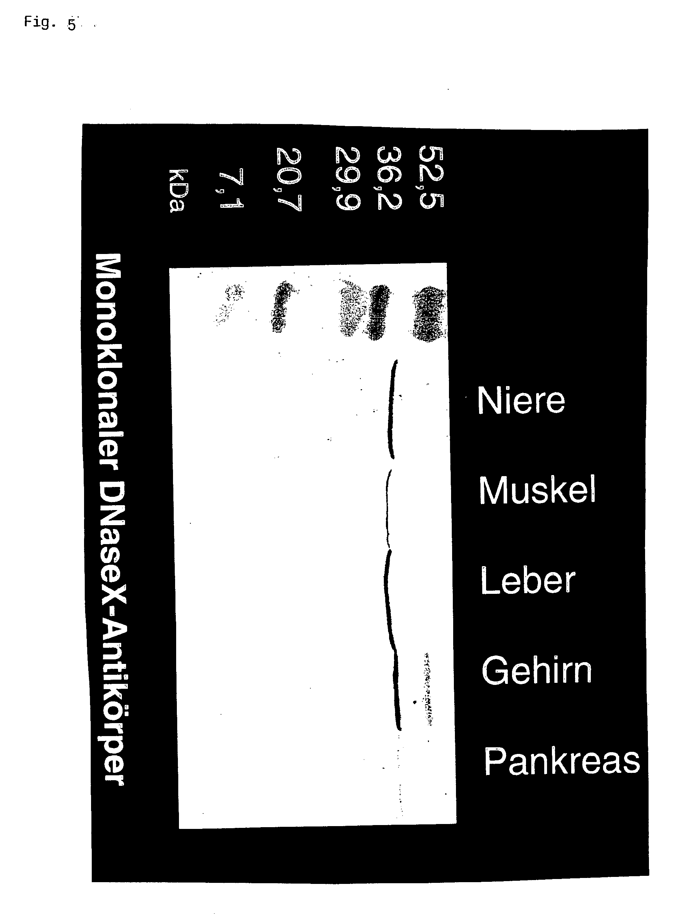

### Fig. 5: Immunhistochemische Analyse des murinen DNase X-Proteins

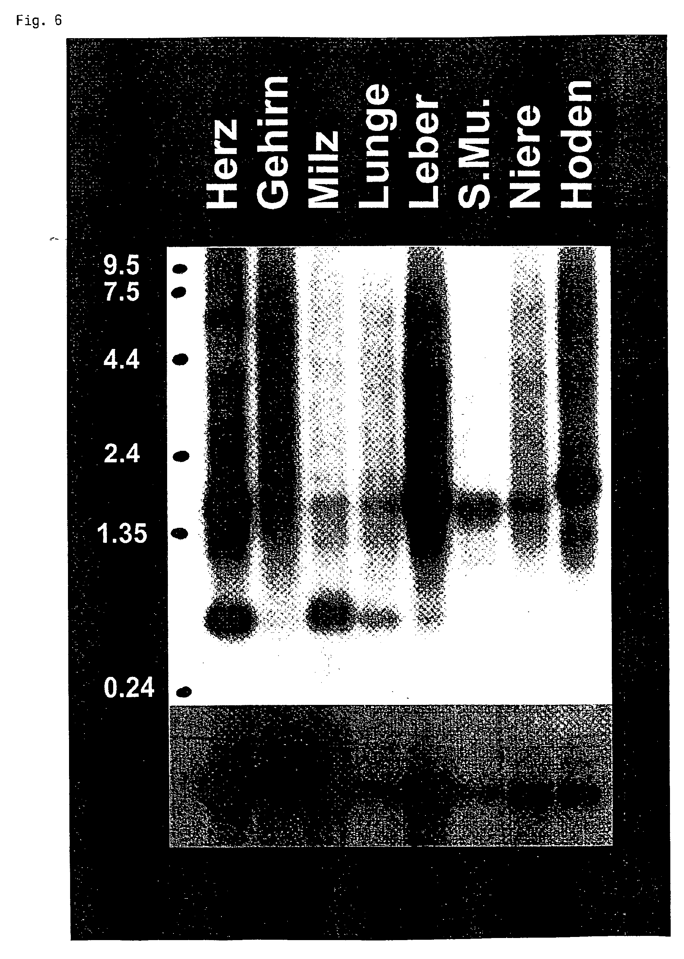

### Fig. 6: Northern Blot

Expression des DNase X-Gens in murinem adultem Gewebe
SMu = Skelettmuskel
Als Vergleich wurde eine murine Transferrinprobe hybridisiert. Sie zeigt die Menge der aufgetragenen RNA pro Spur.

Die folgenden Beispiele erläutern die Erfindung.

### Beispiele

Hinsichtlich der verwendeten Methoden wird auch auf Sambrook, J., Fritsch, E.F. und Maniatis, T. (Molecular cloning; a laboratory manual; second edition; Cold Spring Harbor Laboratory Press, 1989) und Current Protocols in Molecular Biology (John Wiley and Sons, 1994-1998) hingewiesen, wobei die nachfolgend erwähnten Techniken, insbesondere Präparation von DNA bzw. RNA oder Northem-Blot dem Fachmann hinreichend bekannt sind und beherrscht werden.

### Beispiel 1

### Isolierung und Charakterisierung der murine DNase X kodierenden cDNA bzw. genomischen DNA

Die humane DNase X cDNA (JFC4 - Accession Number X90392) wurde radioaktiv markiert und mit einer murinen fötalen Gehirn-cDNA-Bibliothek (Fa. Stratagene, La Jolla) und einer totalen genomischen murinen Cosmid-Bibliothek (Resourcenzentrum Berlin, RZPD) hybridisiert. Die Stringenz der Hybridisierung war auf 50°C reduziert.

Die DNA-Proben wurden mit α-³²P-dATP und α-³²P-dCTP (3000 ci/mmol) in einer random-primed Reaktion markiert (Feinberg und Vogelstein, Anal. Bichem. 132, S. 6-13 (1983)). Die Hybridisierungen wurden in 0,5 M Na-Phosphat, 7% SDS, 0,2% BSA, 0,2% PEG 6000, 0,05% Polyvinylpyrrolidone 360000, 0,05% Ficoll 70000 und 0,5% Dextransulfat bei 50°C über Nacht durchgeführt. Die nicht-gebundene Probe wurde durch Waschen bei 50°C in 40 mM Na-Phosphat, pH 7,2,1% SDS für 60 Minuten entfernt. Die Signale auf den Membranen wurden bei 80°C durch Röntgenfilme (XAR-5, Fa. Kodak) detektiert.

Ein Klon, der die gesamte murine genomische Sequenz des DNaseX-Gens beinhaltet, wurde bei der DSMZ (Deutsche Sammlung von Mikroorganismen und Zellkulturen GmbH, Mascheroder Weg 1b, Braunschweig) gemäß Budapester Vertrag am 2. März 2001 hinterlegt: E. coli JFC-C491 DSM 14141

### Beispiel 2

### Herstellung von Antikörpern

Mit einen synthetisch hergestellten Peptid der Sequenz "CAS LTK KRL DKL ELR TEP GF" werden Tiere zur Erzeugung von Antikörpern gegen murine DNase X wie folgt immunisiert:

### Immunisierungsprotokoll für polyklonale Antikörper im Kaninchen

Pro Immunisierung werden 600 µg gereinigtes KLH-gekoppeltes Peptid in 0,7 ml PBS und 0,7 ml komplettem bzw. inkomplettem Freund's Adjuvans eingesetzt.
- Tag O:: 1. Immunisierung (komplettes Freund's Adjuvans)
- Tag 14:: 2. Immunisierung (inkomplettes Freund's Adjuvans; icFA)
- Tag 28:: 3. Immunisierung (icFA)
- Tag 56:: 4. Immunisierung (icFA)
- Tag 80:: Ausbluten

Das Serum des Kaninchens wird im Immunoblot getestet. Hierzu wird das zur Immunisierung eingesetzte Peptid einer SDS-Polyacrylamid-Gelelektrophorese unterzogen und auf ein Nitrocellulosefilter übertragen (vgl. Khyse-Andersen, J., J. Biochem. Biophys. Meth. 10, (1984), 203-209). Die Western Blot-Analyse wurde wie in Bock, C.-T. et al., Virus Genes 8, (1994), 215-229, beschrieben, durchgeführt. Hierzu wird das Nitrocellulosefilter eine Stunde bei 37°C mit einem ersten Antikörper inkubiert. Dieser Antikörper ist das Serum des Kaninchens (1:10000 in PBS). Nach mehreren Waschschritten mit PBS wird das Nitrocellulosefilter mit einem zweiten Antikörper inkubiert. Dieser Antikörper ist ein mit alkalischer Phosphatase gekoppelter monoklonaler Ziege Anti-Kaninchen-IgG-Antikörper (Dianova) (1:5000) in PBS. Nach 30-minütiger Inkubation bei 37°C folgen mehrere Waschschritte mit PBS und anschließend die alkalische Phosphatase-Nachweisreaktion mit Entwicklerlösung (36µM 5' Bromo-4-chloro-3-indolylphosphat, 400µM Nitroblau-tetrazolium, 100mM Tris-HCI, pH 9.5, 100 mM NaCI, 5 mM MgCl₂) bei Raumtemperatur, bis Banden sichtbar werden.

Es zeigt sich, daß erfindungsgemäße, polyklonale Antikörper hergestellt werden können.

### Immunisierungsprotokoll für polyklonale Antikörper im Huhn

Pro Immunisierung werden 100 µg gereinigtes KLH-gekoppeltes Peptid in 0,8 ml PBS und 0,8 ml komplettem bzw. inkomplettem Freund's Adjuvans eingesetzt.
- Tag O.: 1. Immunisierung (komplettes Freund's Adjuvans)
- Tag 28:: 2. Immunisierung (inkomplettes Freund's Adjuvans; icFA)
- Tag 50:: 3. Immunisierung (icFA)

Aus Eigelb werden Antikörper extrahiert und im Western Blot getestet. Es werden erfindungsgemäße, polyklonale Antikörper nachgewiesen.

### Immunisierungsprotokoll für monoklonale Antikörper der Ratte

Pro Immunisierung werden 250 µg gereinigtes KLH-gekoppeltes Peptid in 0,25 ml PBS und 0,25 ml komplettem bzw. inkomplettem Freund's Adjuvans eingesetzt; bei der 4. Immunisierung ist das Peptid in 0,5 ml (ohne Adjuvans) gelöst.
- Tag O.: 1. Immunisierung (komplettes Freund's Adjuvans)
- Tag 28:: 2. Immunisierung (inkomplettes Freund's Adjuvans; icFA)
- Tag 56:: 3. Immunisierung (icFA)
- Tag 84:: 4. Immunisierung (PBS)
- Tag 87:: Fusion

Überstände von Hybridomen werden im Western Blot getestet. Erfindungsgemäße, monoklonale Antikörper werden nachgewiesen. Einer dieser Antikörper ist der nachfolgend verwendete monoklonale Ratten-Antikörper JFC-DNX6.

### Beispiel 3

### DNase X wird in Zellen mit hohem Turnover überexprimiert

Um die Verteilung des DNaseX-Proteins in der Maus zu untersuchen, wurden Embryonen zu verschiedenen Stadien (E13,5; E 15,5; E17,5) entnommen und in 4% Paraformaldehyd über Nacht fixiert. Danach fand eine Fixierung über Nacht in 75% Ethanol statt. Die weiteren Fixierungsschritte waren: 2 Stunden in 85% Ethanol, 2 Stunden in 96% Ethanol, 2 Stunden in Ethanol absolut, dann über Nacht in Xylol, dann zweimal 2 Stunden in Xylol und dann in Paraffin (58°C) über Nacht, dann zweimal 2 Stunden in Paraffin. Dann werden die Embryonen in Plastikschälchen eingebettet, zum Abkühlen auf eine Kühlplatte gestellt und dann 6µm Schnitte angefertigt. Die Schnitte werden in einer absteigenden Alkoholreihe (2 x 10 Min. Xylol, 2 x 5 Min. 100% EtOH, je 1 x 4 Min. 95%, 80%, 60%, 30% Ethanol, dann Wasser) entparaffiniert.

Die DNase X-Epitope werden freigelegt, indem die Paraffinschnitte in Citratpuffer gekocht werden. Dazu wird 10x Citratpuffer (Antigen Retrival Citra, Fa. BioGenex, San Ramon, CA 94583) 1:10 verdünnt und die Schnitte werden in eine hitzeresistente Plastikbox einsortiert. Darin werden die Schnitte in der Mikrowelle 2 Min. auf Maximun und 8 Min. auf 180°C erhitzt. Danach läßt man die Schnitte 30 Minuten abkühlen und gibt sie dann 5 Min. in PBS-Lösung. Auf jeden Objektträger werden 400 ml Peroxidase-Blocklösung geschüttet und 40 Minuten bei Zimmertemperatur in einer Kammer inkubiert. Die Objektträger werden 10 Minuten mit PBS zum Spülen inkubiert und danach trockengewischt. Der Erstantikörper (JFC-DNX6) wird mit PBS 1:100 verdünnt und 300 ml dieser Antikörper-Lösung werden auf jeden Objektträger gegeben und darin über Nacht im Kühlschrank in einer Kammer inkubiert. Die Objektträger werden 10 Minuten mit PBS gespült. Sie werden trockengewischt und 300 ml des in PBS 1:100 verdünnten Zweitantikörpers (Alkaline Phosphatase conjugated AffiniPure Goat Anti-Rat IgG+IgM (H+L), Jackson ImmunoResearch Laboratories, Inc.) werden auf jeden Objektträger gegeben. Die Inkubation erfolgt 45 Minuten bei Zimmertemperatur in einer Kammer. Danach wird mit PBS gespült und die Objekträger 10 Minuten in PBS gebadet. Die Objektträger werden trockengewischt. Danach wird eingefärbt mit frisch angesetztem Färbepuffer A (100 mM Tris-HCI, pH 9,5, 100 mM NaCI, 5 mM MgCl₂ x 6 H₂O). Man verwendet für die Färbung 10 ml des Färbepuffers A. Dazu gibt man dann 33 µl BZip (5-Bromo-4-Chloro-3-lndolyl-Phosphatase) (50 mg/2 ml Dimethylformamid) und 66 µl NBT (NitroBlue Tetrazolium) (10 mg/200 µl Dimethylformamid). Es wird gut gemischt und dunkel gestellt. Pro Objekträger werden 300 µl Flüssigkeit verwendet. Es wird 2 Minuten inkubiert, danach mit Wasser gespült und die Objektträger werden einzeln für 30 Sec. in frisch filtriertes Hämatoxilin gestellt. Dann mit Wasser einzeln gespült bis keine Farbe mehr kommt. Danach werden die Objektträger luftblasenfrei mit Eukitt eingedeckt und die lichtmikroskopische Analyse durchgeführt.

Das Ergebnis ist in Fig. 4 A, B, C zu sehen.

Eine weitere immunhistochemische Untersuchung wurde an erwachsenen Mausgeweben durchgeführt. Die Durchführung des Experiments war analog wie vorstehend beschrieben. Es wurde nachgewiesen, daß murines DNase X-Protein in Niere, Muskel, Leber, Gehirn und Pankreas der Maus vorkommt (Fig. 5).

### Beispiel 4

### Northern Blot

Die 'multiple tissue Northern blots' wurden von der Firma CLONTECH (Palo Alto, Kalifornien, USA) gekauft und nach den Anweisungen des Herstellers angewendet. Die DNA-Proben von DNase X (Bp 300 bis Bp 760 der Spleißform 1, s. Fig. 2a) wurde radioaktiv markiert und mit den Northem hybridisiert. Zur Markierung doppelsträngiger DNA wurde die "random priming" Methode verwendet.

### a) Random Priming:

Für einen typischen Markierungsansatz werden 100 ng DNA in einem Volumen von 12 µl gelöst. 10 minütiges Erhitzen auf 95°C bewirkt die Denaturierung der DNA in Einzelstränge. Der Ansatz wird auf Eis gelagert, um eine Reassoziation der beiden komplementären DNA-Stränge zu verhindern. Den Reaktionsansatz durch 4 µl OLB, 1 µl Klenow (1U) sowie 2,5 µl a-³²P- dCTP und 2,5 µl a-³²P-dATP komplettieren. Über Nacht bei Raumtemperatur inkubieren. Während dieser Zeit findet die Bildung des Komplementärstranges, ausgehend von den an einen Einzelstrang angelagerten Hexanucleotiden, durch das Klenow-Fragment der *E. coli* DNA-Polymerase I statt. Die radioaktive Markierung der DNA erfolgt durch den Einbau des α-³²PdCTP und des α-³²P-dATP.

Die Abtrennung der nichteingebauten Nucleotide erfolgte mit Hilfe einer selbst gefertigten Sephadex G-50 Säule. Das Auftrennungsprinzip der Säule beruht auf der Ausschlußchromatographie. Die kleineren nichteingebauten Nucleotide passen in kleine Poren des Säulenmaterials, während die DNA von diesen ausgeschlossen bleibt. Das Volumen, in dem sich die Nucleotide bewegen können ist daher größer als das Volumen, das der DNA zur Verfügung steht. Trägt man nun ein Gemisch aus DNA und Nucleotiden auf die Säule, so läuft die DNA schneller als die Nucleotide durch die Säule. Dies erlaubt die Abtrennung der nichteingebauten Nucleotide. Dazu wird eine Pasteurpipette mit einem kleinen Glaskügelchen verschlossen. Auffüllen der Pasteurpipette mit in Wasser gelöstem Sephadex G-50 ("Fine") bis sich das Füllmaterial 5 cm unter der Oberkante der Pasteurpipette befindet. 2x Spülen der Säule mit TE. Auftragen des radioaktiven Markierungsansatzes. Zugabe von 320 µl TE. Die Lösung, die durch die Säule gelaufen ist, verwerfen. Eppendorf-Tube unter die Säule stellen. Zugabe von 350 µl TE. Auffangen der durch die Säule gelaufenen radioaktiven Lösung.

### b) Hybridisisierung:

Die Hybridisierung der Northern Blots erfolgte wie nachfolgend beschrieben. Zunächst wurden die Northern blots in 10 ml Hybridisierungslösung (350 ml 20%iges SDS, 500 ml 1M Phosphatpuffer, pH 7,2; 150 ml dest. Wasser) bei 65°C prähybridisiert. Dazu die wurden Northern blots in einer Glasröhre in einem Hybridisierungs-Rollofen für die Dauer von 6 h bei 65°C inkubiert.

Die Prähybridisierungslösung wurde verworfen. Die radioaktiv markierte Probe wurde mit 10 ml Hybridisierungslösung (65°C) auf die Filter gegeben.

Die Hybridisierung erfolgte über Nacht bei 65°C. Die Filter wurden dann zweimal 30 min mit etwa 500 ml Waschpuffer (80 ml 1M Phosphatpuffer, pH 7,2; 100 ml 20% iges SDS, 1820 ml dest. Wasser) im Schüttelbad bei 65°C gewaschen.

### c) Autoradiographie:

Die Filter wurden in Kunststoffolie eingeschweißt. Die Autoradiographie erfolgte bei -80°C in einer Röntgenkassette, die eine Verstärkerfolie aus Calciumwolframat enthielt. Die Exponierung dauerte je nach Stärke des Signals 1-4 Tage.

Die Ergebnisse der durchgeführten Northern Blots sind in Fig. 6 gezeigt.

## Patentansprüche

1. DNA-Sequenz, die murine DNase X mit der in Figur 2 a, b oder c gezeigten Aminosäuresequenz kodiert.

2. DNA-Sequenz, die die in Figur 1 gezeigte DNA-Sequenz umfaßt.

3. DNA-Sequenz, die ein Protein mit den biologischen Eigenschaften einer DNase X kodiert, die ein Fragment, eine allelische Variante oder eine andere Variante der DNA-Sequenz von Anspruch 1 oder 2 ist.

4. Expressionsvektor, der eine DNA-Sequenz nach einem der Ansprüche 1 bis 3 enthält.

5. Murines DNase X-Protein oder ein Protein mit dessen biologischer Aktivität, das von der DNA-Sequenz nach einem der Ansprüche 1 bis 3 kodiert wird.

6. Antikörper, der spezifisch für das Protein von Anspruch 5 ist.

7. Diagnoseverfahren zum Nachweis einer veränderten Expression oder Konzentration von DNase X, bei dem man eine Probe mit einer zur spezifischen Hybridisierung mit einer von der DNA-Sequenz nach einem der Ansprüche 1 bis 3 transkribierten mRNA geeigneten Sonde, einem Primer oder Primersatz oder einem Antikörper gemäß Anspruch 6 in Berührung bringt und sodann direkt oder indirekt bestimmt, ob sich die Konzentration von DNase X-mRNA oder DNase X-Protein in der Probe im Vergleich zu einer Kontrollprobe unterscheiden.

8. Diagnostischer Kit zur Durchführung des Verfahrens nach Anspruch 7, der eine zur spezifischen Hybridisierung mit einer von der DNA-Sequenz nach einem der Ansprüche 1 bis 3 transkribierten mRNA geeignete Sonde, einen Primer oder Primersatz und/oder einen Antikörper nach Anspruch 6 enthält.

9. Arzneimittel, das eine DNA-Sequenz nach einem der Ansprüche 1 bis 3, einen Expressionsvektor nach Anspruch 4 oder ein DNase X-Protein nach Anspruch 5 enthält.

10. Verwendung einer in einem der Ansprüche 1 bis 5 definierten Verbindung zur Prävention und/oder Therapie von Erkrankungen, bei denen Apoptose eine Rolle spielt.

11. Verwendung nach Anspruch 10, wobei die Erkrankungen systemischer Lupus Erythematosus (SLE), AIDS, Krebserkrankungen, zytische Fibrose oder Atrophie der Prostata sind.

12. Nicht-menschliches Säugetier, **dadurch gekennzeichnet, daß** das eine DNA-Sequenz nach einem der Ansprüche 1 bis 3 umfassende DNase X-Gen ubiquitär oder gewebespezifisch verändert ist.

13. Nicht-menschliches Säugetier nach Anspruch 12, wobei das DNase X-Gen inaktiviert ist.

14. Verfahren zur Herstellung des nicht-menschlichen Säugetiers nach Anspruch 11 oder 12, das folgende Schritte umfaßt:
(a) Herstellung eines DNA-Fragments, insbesondere eines Vektors, der ein verändertes murines DNase X-Gen enthält, wobei das DNase X-Gen durch Insertion einer heterologen Sequenz, insbesondere eines selektierbaren Markers, verändert worden ist;
(b) Präparation embryonaler Stammzellen aus einem nicht-menschlichen Säuger;
(c) Transformation der embryonalen Stammzellen von Schritt (b) mit dem DNA-Fragment von Schritt (a), wobei das DNase X-Gen in den embryonalen Stammzellen durch homologe Rekombination mit dem DNA-Fragment von (a) verändert wird;
(d) Kultivieren der Zellen von Schritt (c),
(e) Selektion der kultivierten Zellen von Schritt (d) auf das Vorhandensein der heterologen Sequenz, insbesondere des selektierbaren Markers,
(f) Erzeugen chimärer nicht-menschlicher Säuger aus den Zellen von Schritt (e) durch Injektion dieser Zellen in Säuger-Blastozysten; und
(g) Übertragen der in Schritt (f) erhaltenen Blastozysten in pseudo-schwangere weibliche Säuger und Analyse der erhaltenen Nachkommen hinsichtlich eines veränderten, insbesondere inaktivierten DNase X-Gens.

15. Verwendung des nicht-menschlichen Säugetiers nach Anspruch 12 oder 12 oder des nach einem Verfahren nach Anspruch 14 erhältlichen nicht-menschlichen Säugetiers zur weiteren Erforschung von SLE, AIDS, Tumorerkrankungen und Zystischer Fibrose oder zur Charakterisierung von Genen und/oder Testung von Substanzen, Arzneimitteln und/oder Therapieansätzen, die mit diesen Erkrankungen oder zu diesen Erkrankungen führenden Prozessen in Zusammenhang stehen.

16. Nicht-menschliches Säugetier nach Anspruch 12 oder 13, Verfahren nach Anspruch 14 oder Verwendung nach Anspruch 15, wobei das nicht-menschliche Säugetier eine Maus ist.
